# EUROPEAN PATENT SPECIFICATION

(11) **EP 2 373 804 B1**
(45) Date of publication and mention of the grant of the patent: **12.11.2014**
(21) Application number: 09831242.4
(22) Date of filing: 04.12.2009
(51) Int. Cl.: C12P 19/34, C12Q 1/68

(54) **COMPOSITIONS FOR IMPROVING GENE AMPLIFICATION**
ZUSAMMENSETZUNGEN FÜR VERBESSERTE GENAMPLIFIKATION
COMPOSITIONS POUR AMÉLIORER L'AMPLIFICATION GÉNIQUE

(30) Priority: 16.03.2009 US 160606 P; 05.12.2008 US 201052 P
(43) Date of publication of application: 12.10.2011
(73) Proprietor: DNA Polymerase Technology, Inc., St. Louis, MO 63104 (US)
(72) Inventor: ZHANG, Zhian, Ballwin MO 63021 (US); KERMEKCHIEV, Milko, University City MO 63124 (US)
(74) Representative: Goodall, Scott
(86) International application number: PCT/US2009/066868
(87) International publication number: WO 2010/065924

(56) References cited:
- US-A1- 2004 265 969
- US-A1- 2007 219 366
- MARCO MUSSO ET AL: "Betaine, Dimethyl Sulfoxide, and 7-Deaza-dGTP, a Powerful Mixture for Amplification of GC-Rich DNA Sequences", THE JOURNAL OF MOLECULAR DIAGNOSTICS, vol. 8, no. 5, 1 November 2006 (2006-11-01), pages 544-550, XP055043399, ISSN: 1525-1578, DOI: 10.2353/jmoldx.2006.060058
- RALSER M ET AL: "An efficient and economic enhancer mix for PCR", BIOCHEMICAL AND BIOPHYSICAL RESEARCH COMMUNICATIONS, ACADEMIC PRESS INC. ORLANDO, FL, US, vol. 347, no. 3, 1 September 2006 (2006-09-01), pages 747-751, XP024925566, ISSN: 0006-291X, DOI: 10.1016/J.BBRC.2006.06.151 [retrieved on 2006-09-01]
- ROSEANU ET AL.: 'Inhibition of Binding of Lactoferrin to the Human Promonocyte Cell Line THP- 1 by Heparin: the Role of Cell Surface Sulphated Molecules.' BIOCHIM BIOPHYS ACTA vol. 1475, no. 1, June 2000, pages 35 - 38, XP004276588
- BURCKHARDT ET AL.: 'Amplification of DNA from Whole Blood.' GENOME RES. vol. 3, no. 4, February 1994, pages 239 - 243, XP008166946

## Description

### STATEMENT REGARDING FEDERALLY SPONSORED RESEARCH OR DEVELOPMENT

This invention was made in part with Government support under R44 GM073401 NIH. The Government has certain rights in the invention.

### INCORPORATION-BY-REFERENCE OF SEQUENCE LISTING

The Sequence Listing, which is a part of the present disclosure, includes a computer readable form comprising nucleotide and/or amino acid sequences of the present invention.

### FIELD OF THE INVENTION

The present invention generally relates to enhancement of direct polynucleotide detection amplificaiton reactions, such as conventional PCR, RT-PCR, real-time PCR, real-time RT PCR, and sequencing, especially, for crude sample.

### BACKGROUND

The success and sensitivity of DNA detection in important clinical, diagnostic and forensic applications of PCR of blood specimens is limited by the presence of blood inhibitors of Taq polymerase, such as the heme, IgG fractions, and other blood components. Plain Taq enzyme can be completely inhibited by 0.004%-0.2% blood (vol/vol) (Al-Soud WA, Radstrom P. 2000. Effect of amplification facilitators on diagnostic PCR in the presence of blood, feces and meat. J. Clin. Microbiol. 38: 4463-70; Al-Soud AW, Jönsson LJ, Radstrom P. 2000. Identification and characterization of immunoglobulin G in blood as a major inhibitor of diagnostic PCR. J. Clin. Microbiol. 38:345-50; Al-Soud AW, Radstrom P. 1998. Capacity of nine thermostable DNA polymerases to mediate DNA amplification in the presence of PCR-inhibiting samples. Appl. Environ. Microbiol. 64:3748-53). To overcome this inhibition, high cost and additional labor-demanding methods are currently used to purify DNA from blood prior to PCR. Nevertheless, this inhibition is still a serious concern with many PCR-based human blood tests, since even after purifying DNA from the blood, traces of the PCR inhibitors can generate as high as 14% false negative results, as published for hepatitis B blood tests (Kramvis A, Bukofzer S and Kew MC. Comparison of hepatitis B virus DNA extractions from serum by the QIAamp blood kit, GeneReleaser, and the phenol-chloroform method. J Clin Microbiology 1996; 34:2731-2733).

A similar problem exists with environmental samples, including soil and water samples, using PCR mainly due to the inhibitory effects of humic acid (HA) and other inhibitors. Of particular importance is the PCR analysis of soil samples, which includes the microbial balance and dynamic in the environment, and human health related tests for potential viral and microbial infections. Another significant aspect of soil PCR applications is monitoring bio-terrorism activity. For example, fast, sensitive and quantitative analysis of anthrax spores from environmental samples is essential for accurate detection and risk assessment. Trace amounts of humic substances in a PCR reaction can yield a false-negative result or partially inhibit the reaction, thus compromising the sensitivity of detection (Tsai, Y.L. and Olson, B.H. 1992. Rapid method for separation of bacterial DNA from humic substances in sediments for polymerase chain reaction. Appl. Environ. Microbiol., 58, 2292-2295; Watson, R.J. and Blackwell, B. 2000. Purification and characterization of a common soil component which inhibits the polymerase chain reaction. Can. J. Microbiol., 46, 633-642). Some studies suggest that the inhibitory effect of humic acid on PCR, which co-extracts with DNA, is associated with direct inactivation of the thermostable DNA polymerase (Moreira, D. 1998. Efficient removal of PCR inhibitors using agarose-embedded DNA preparations. Nucleic Acids Res., 26, 3309-3310).

It has been reported that ostomoprotectants such as Betaine, Proline and L-carnitine (see e.g., US Pat No 6,787,305), and non-ionic detergent NP-40 (see e.g., US Pat No 5,766,890) can enhance PCR reaction in purified DNA samples. D-(+)-trehalose can thermostabilize and thermoactivate thermolabile enzymes (Carninci et al. 1998 Proc. Natl. Acad. Sci. U. S. A, 95, 520-524).

An inhibitory effect of heparin on Taq in PCR with purified DNA or RNA substrate has been documented (see e.g., Chen et al. 1990 Nucleic Acids Res 18, 3255-3260; Satsangi et al. 1994 Lancet 343, 1509-1510; Poli et al. 1993 PCR Methods Appl. 2, 356-8; Wang et al. 1992 J Clin Microbiol. 303, 750-753). It has been reported that all three commonly used anticoagulants EDTA, citrate, and heparin inhibit amplification reactions in anticoagulant-treated whole blood; citrate-treated blood required an unusually high salt buffer for PCR; leukocytes in EDTA-treated blood were difficult to lyse; and that heparin is inhibitory but less so than EDTA or citrate in whole blood (Burckhardt J: Amplification of DNA from whole blood. PCR Methods Appl 1994, 3: 239-243). But to date there has been no report of an enhancing effect of heparin in either whole blood or crude blood samples. Nor has there been a report that heparin can bind or potentially deactivate any known major PCR inhibitor, such as lactoferrin or serum IgG fraction. In fact, heparin is widely understood to be inhibitory in PCR generally.

There is a demand for efficient facilitator/enhancer additives which could relieve the inhibition of Taq DNA polymerase in PCR and improve the yield of the amplified product. However, the existing additives are usually restricted to certain functions, such as lowering the melting temperature of DNA or resolving secondary structure of the template, and most work only on substantially purified DNA (Kang, J., Lee, M.S. and Gorenstein, D.G. 2005. The enhancement of PCR amplification of a random sequence DNA library by DMSO and betaine: application to in vitro combinatorial selection of aptamers. J. Biochem. Biophys. Methods, 64, 147-151; Schnoor, M., Voss, P., Cullen, P., Boking, T., Galla, H.J., Galinski, E.A. and Lorkowski, S. 2004. Characterization of the synthetic compatible solute homoectoine as a potent PCR enhancer. Biochem. Biophys. Res. Commun., 322, 867-872; Chakrabarti, R. and Schutt, C.E. 2002. Novel sulfoxides facilitate GC-rich template amplification. Biotechniques, 32, 866, 868, 870-2, 874; Chakrabarti, R. and Schutt, C.E. 2001. The enhancement of PCR amplification by low molecular-weight sulfones. Gene, 274, 293-298; Chakrabarti, R. and Schutt, C.E. 2001. The enhancement of PCR amplification by low molecular weight amides. Nucleic Acids Res., 29, 2377-2381; Al-Soud, W. and Radstrom, P. 2000. Effects of amplification facilitators on diagnostic PCR in the presence of blood, feces, and meat. J. Clin. Microbiol., 38, 4463-4470; Henke, W., Herdel, K., Jung, K., Schnorr, D. and Loening, S.A. 1997. Betaine improves the PCR amplification of GC-rich DNA sequences. Nucleic Acids Res., 25, 3957-3958; Musso, M., Bocciardi, R., Parodi, S., Ravazzolo, R. and Ceccherini, I. 2006. Betaine, dimethyl sulfoxide, and 7-deaza-dGTP, a powerful mixture for amplification of GC-rich DNA sequences. J. Mol. Diagn., 8, 544-550; Ralser, M., Querfurth, R., Warnatz, H.J., Lehrach, H., Yaspo, M.L. and Krobitsch, S. 2006. An efficient and economic enhancer mix for PCR. Biochem. Biophys. Res. Commun., 347, 747-751). Furthermore, conventional additives, such as DMSO or formamide can inhibit various DNA polymerases (Varadaraj, K. and Skinner, D.M. (1994) Denaturants or cosolvents improve the specificity of PCR amplification of a G + C-rich DNA using genetically engineered DNA polymerases. Gene 140, 1-5).

As an alternative to the various time and labor consuming pre-PCR procedures with blood and soil samples, there exists mutant(s) of Taq DNA polymerase with resistance to PCR inhibitors in blood and soil sample (see WO/2008/034110).

### SUMMARY OF THE INVENTION

Briefly, the present invention is directed to an enhancer mixture which improves the performance of polymerase enzymes in amplification reactions, especially under higher concentrations of PCR inhibitors or against more difficult targets, such as those with high GC content.

One aspect of the invention provides a gene amplification enhancer composition comprising trehalose, carnitine, and a non-ionic detergent. In some embodiments, the enhancer composition further comprises heparin.

Concentrations recited below refer to concentration per amplification reaction mixture volume, except for heparin concentrations which refer to concentration per volume of blood or blood fraction in the amplification reaction mixture.

In some embodiments, the enhancer composition comprises about 0.1 to about 1.0 M D-(+)-trehalose; 0.1 to 1.5 M L-carnitine; 0.1% to 8% non-ionic detegent; and, optionally, 2 to 50 units heparin per mL of whole blood, plasma, or serum in the reaction mixture.

In various embodiments, the enhancer composition comprises 0.1 to 1.0 M; 0.2 M to 1.0 M; 0.3 M to 0.9 M; 0.4 to 0.8 M; or 0.5 M to 0.7 M trehalose. In some embodiments, the enhancer composition comprises about 0.6 M trehalose.

In various embodiments, the enhancer composition comprises 0.1 M to 1.5 M; 0.1 M to 1.4 M; 0.1 M to 1.3 M; 0.1 M to 1.2 M; 0.1 M to 1.1 M; 0.1 M to 1.0 M; 0.1 M to 1.0 M; 0.1 M to 1.0 M; 0.2 M to 0.8 M; 0.3 M to 0.7 M; or 0.4 M to 0.6 M carnitine. In some embodiments, the enhancer composition comprises about 0.5 M carnitine.

In various embodiments, the enhancer composition comprises 0.01 % to 8% non-ionic detergent; 0.02% to 7.5% non-ionic detergent; 0.03% to 7% non-ionic detergent; 0.04% to. 6.5% non-ionic detergent; 0.05% to 6% non-ionic detergent; 0.06% to 5.5% non-ionic detergent; 0.07% to 5% non-ionic detergent; 0.08% to 4.5% non-ionic detergent; 0.09% to 4% non-ionic detergent; 0.1 % to 3.5% non-ionic detergent; 0.1% to 3% non-ionic detergent; 0.1% to 2.5% non-ionic detergent; 0.1 % to 2% non-ionic detergent; 0.1% to 1.5% non-ionic detergent; 0.1% to 1.4% non-ionic detergent; 0.1% to 1.3% non-ionic detergent; 0.1% to 1.2%; 0.1% to 1.1%; 0.2% to 1.0%; 0.3% to 0.9%; or 0.4% to 0.8% per amplification reaction mixture volume. In some embodiments, the non-ionic detergent is Brij-58, NP-40, Nonidet P-40, Igepal CA-630, Brij-58, Tween-20, NP-40, and Triton X-100. In some embodiments, the non-ionic detergent is a polyoxyethylene cetyl ether, such as Brij-58, at a concentration of 0.01 % to 4.0%; 0.02% to 1.0%; 0.03% to 0.5%; or 0.04% to 0.2% per amplification reaction mixture volume. In some embodiments, the non-ionic detergent is a nonyl phenoxylpolyethoxylethanol, such as NP-40, at a concentration of 0.1 % to 1.1 %; 0.2% to 1.0%; 0.3% to 0.9%; or 0.4% to 0.8% per amplification reaction mixture volume.

In various embodiments, the enhancer composition comprises 2 units to 50 units; 5 units to 45 units, 5 units to 40 units, 5 units to 35 units, 5 units to 30 units, 5 units to 25 units, 5 units to 15 units, 6 units to 14 units, 7 units to 13 units, 8 units to 12 units, or 9 units to 11 units of heparin per mL of whole blood, plasma, or serum, where present in the reaction mixture or assay mixture. In some embodiments, the enhancer composition comprises 10 units of heparin per mL plasma or serum, where present in the reaction mixture or assay mixture.

Another aspect of the invention provides a method of amplifying a target nucleic acid in a polymerase chain reaction (PCR) using an enhancer composition described herein. In various embodiments, the method comprises forming an assay mixture comprising a sample comprising a target nucleic acid; at least one polymerase; and an enhancer composition described herein; and amplifying the target nucleic acid in the assay mixture.

In some embodiments, the PCR is a real-time PCR. In some embodiments, the PCR is a reverse-transcriptase (RT) PCR. In some embodiments, the PCR is a real-time RT-PCR

In various embodiments, the target nucleic acid comprises a DNA molecule. In various embodiments, the target nucleic acid comprises an RNA molecule. In some embodiments, the PCR is a reverse-transcriptase (RT) PCR; the target nucleic acid comprises an RNA molecule; and the assay mixture further comprises a reverse-transcriptase.

In various embodiments, the sample comprises a heterogeneous mixture of nucleic acid targets of varying G+C content. In some embodiments, the heterogeneous mixture of nucleic acid templates comprises targets comprising 30% to 81 % G+C content. In some embodiments, the heterogeneous mixture of nucleic acid templates comprises one or more of endogenous DNA, endogenous RNA, exogenous DNA, or exogenous RNA. In some embodiments, the endogenous DNA is cellular DNA; the endogenous RNA is cellular RNA; the exogenous DNA is pathogen (e.g., viral) DNA; or the exogenous RNA is pathogen (e.g., viral) RNA.

In various embodiments, the assay mixture comprises a volume of whole blood, blood fraction, or a combination thereof. In some embodiments, the assay mixture comprises a volume of whole blood, plasma, serum, or a combination thereof. In some embodiments, the whole blood, plasma, serum, or combination thereof is at least 1% up to 25%; at least 3% up to 20%; or at least 5% up to 15% of a total volume of the assay mixture.

In various embodiments, the assay mixture comprises a volume of soil or soil extract. In some embodiments, the soil or soil extract is at least 1 % up to 90%; at least 1% up to 80%; at least 1 % up to 70%; at least 1% up to 60%; at least 1 % up to 50%; at least 1% up to 40%; at least 1 % up to 30%; at least 1% up to 20%; or at least 1% up to 10% of a total volume of the assay mixture. In some embodiments, the soil or soil extract comprises a humic acid and the soil or soil extract is present in the assay mixture at a soil or soil extract equivalent amount that provides up to 25 ng of humic acid per 50 uL reaction volume; up to 20 ng of humic acid per 50 uL reaction volume; or up to 10 ng of humic acid per 50 uL reaction volume.

In various embodiments, the assay mixture comprises at least one dye. In some embodiments, the at least one dye is at least one fluorescent dye. In some embodiments, the at least one dye is selected from the group consisting of SYBR Green, Ethidium Bromide, PICO, TOTO, YOYO or LC Green. In some embodiments, the dye is present in the assay mixture at least 0.5X up to 256X, up to 128X, up to 64X, or up to 32X, where X is a manufacturer unit for concentration.

In various embodiments, the assay mixture comprises at least one DNA polymerase. In some embodiments, the at least one polymerase comprises a dye-resistant polymerase activity, blood-resistant polymerase activity, or soil-resistant polymerase activity, or a combination thereof. In some embodiments, the at least one polymerase is selected from the group consisting of OmniTaq (FLAC-22), Omni Klentaq (KT-10), Omni Klentaq-LA, wild type Taq; FastStart Taq; JumpStart Taq; HotStart Plus Taq; AmpliTaq Gold, KlenTaq, FL-12, FL-10, and KT-12.

In various embodiments, the assay mixture comprises at least two polymerase. In some embodiments, the assay mixture comprises OmniTaq and Omni Klentaq.

Another aspect of the invention provides a method of amplifying a target nucleic acid from a crude sample in a polymerase chain reaction (PCR) through the addition of an effective amount of heparin. In various embodiments, the method comprises forming an assay mixture that includes (a) a sample, the sample containing at least a target nucleic acid and whole blood, a blood fraction, or a combination thereof; (b) at least one polymerase; and (c) an amount of heparin effective to (i) increase efficiency of PCR or (ii) reduce or eliminate an inhibitory effect of the whole blood, blood fraction, or combination thereof on PCR; and amplifying the target nucleic acid in the assay mixture.

In various embodiments, the assay mixture comprises a blood fraction. In some embodiments, the blood fraction is plasma, serum, or a combination thereof. In some embodiments, the blood fraction is plasma, serum, or a combination thereof, and the amount of heparin is effective to reduce or eliminate the inhibitory effect of the plasma or serum on PCR.

In various embodiments, the the whole blood, blood fraction, plasma, serum, or combination thereof is at least 1% up to25%; at least 3% up to20%; or at least 5% up to 15% of a total volume of the assay mixture.

In various embodiments, the amount of heparin is equivalent to 2 units to 50 units; 5 units to 45 units, 5 units to 40 units, 5 units to 35 units, 5 units to 30 units, 5 units to 25 units, 5 units to 20 units, 5 units to 15 units, 6 units to 14 units, 7 units to 13 units, 8 units to 12 units, or 9 units to 11 units of heparin per mL of whole blood, blood fraction, plasma, serum, or combination thereof in the assay mixture. In some embodiments, the amount of heparin is equivalent to 10 units of heparin per mL of whole blood, blood fraction, plasma, serum, or combination thereof in the assay mixture.

In various embodiments, the target nucleic acid comprises a DNA molecule. In various embodiments, the PCR is a real-time PCR. In various embodiments, the PCR is a reverse-transcriptase (RT) PCR, the target nucleic acid comprises an RNA molecule, and the assay mixture further comprises a reverse-transcriptase. In some embodiments, the RT-PCR is a real-time RT-PCR.

In various embodiments, the sample comprises a heterogeneous mixture of nucleic acid targets of varying G+C content. In some embodiments, the heterogeneous mixture of nucleic acid templates comprises targets comprising 30% to 81 % G+C content. In some embodiments, the heterogeneous mixture of nucleic acid templates comprises one or more of endogenous DNA, endogenous RNA, exogenous DNA, or exogenous RNA. In some embodiments, the endogenous DNA is cellular DNA; the endogenous RNA is cellular RNA; the exogenous DNA is pathogen DNA; or the exogenous RNA is pathogen RNA.

In various embodiments, the assay mixture comprises at least one DNA polymerase. In some embodiments, the at least one polymerase comprises a dye-resistant polymerase activity, blood-resistant polymerase activity, or soil-resistant polymerase activity, or,a combination thereof. In some embodiments, the at least one polymerase is selected from the group consisting of OmniTaq, Omni Klentaq, Omni Klentaq-LA, wild type Taq; FastStart Taq; JumpStart Taq; HotStart Plus Taq; AmpliTaq Gold, KlenTaq, FL-12, FL-10, and KT-12. In some embodiments, the assay mixture comprises at least two polymerases. In some embodiments, the at least two polymerases comprise OmniTaq and Omni Klentaq.

Other objects and features will be in part apparent and in part pointed out hereinafter.

### BRIEF DESCRIPTION OF THE DRAWINGS

Those of skill in the art will understand that the drawings, described below, are for illustrative purposes only. The drawings are not intended to limit the scope of the present teachings in any way.

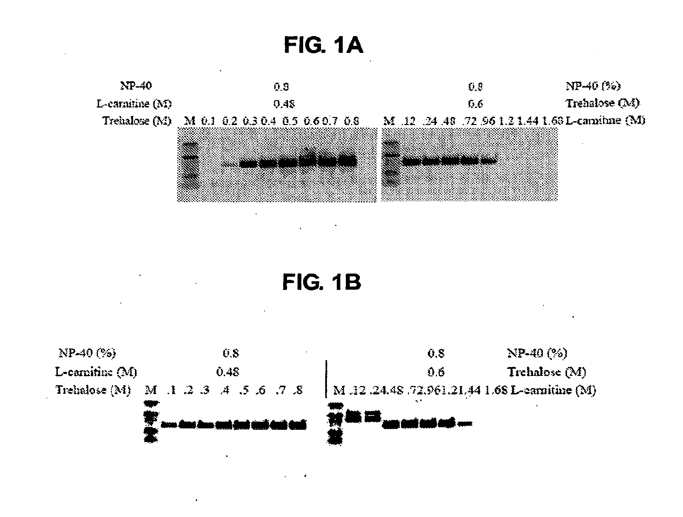

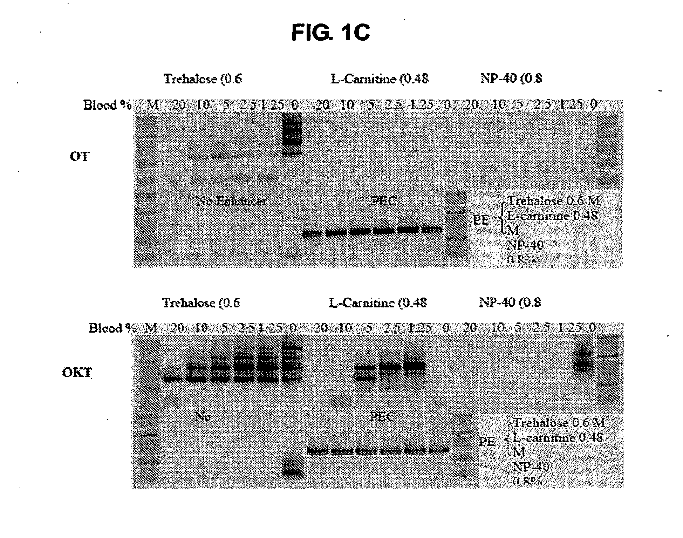

FIG. 1 is a series of agarose gel images depicting the effect of PCR enhancer cocktail (PEC) on amplification of human HIV receptor gene target. FIG. 1A shows the amplification of a 630 bp of human HIV receptor gene target (CCR5, GC content is 47%). FIG. 1B shows the amplification of a 488 bp target of human HTLV-1 related sequence (HRES-1-c, GC content is 81%). PCR was performed directly from 10% blood using OmniTaq (OT) in the presence of different combinations of PEC. In FIG. 1C, the HRES-1-c target was amplified from various blood amounts up to 25%, and the control reaction contained no blood but 4 ng human DNA. OmniTaq and Omni Klentaq (OKT) enzymes were used in the presence of individual components of PEC or a PEC cocktail. The control didn't contain any enhancer (FIG. 1 C). Further details regarding methodology are provided in Example 1.

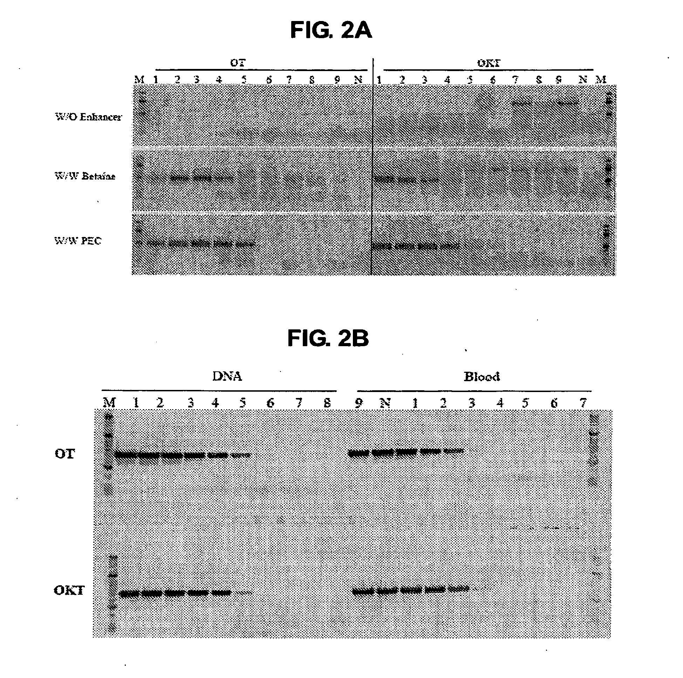

FIG. 2 is a series of agarose gel images depicting the effect of PEC on PCR specificity and sensitivity. FIG. 2A shows the effect of PEC on PCR specificity assessed by amplification of HRES1-1c target directly from blood samples in absence or presence of PEC. FIG. 2B shows that in the presence of PEC the target is amplified both from DNA and blood with similar efficiency. Further details regarding methodology are provided in Example 2.

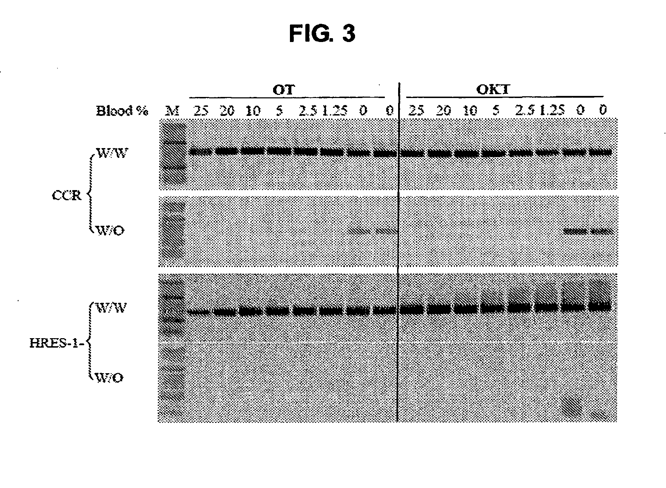

FIG. 3 is a series of agarose gel images depicting the effect of concentration of blood on OmniTaq and Omni Klentaq mutant DNA polymerases. A 630 bp target of the CCR5 gene and a 488 bp target of the HRES-1-c gene were amplified from 0% (4 ng DNA) to 25% blood using OT and OKT in the presence or absence of PEC. Further details regarding methodology are provided in Example 3.

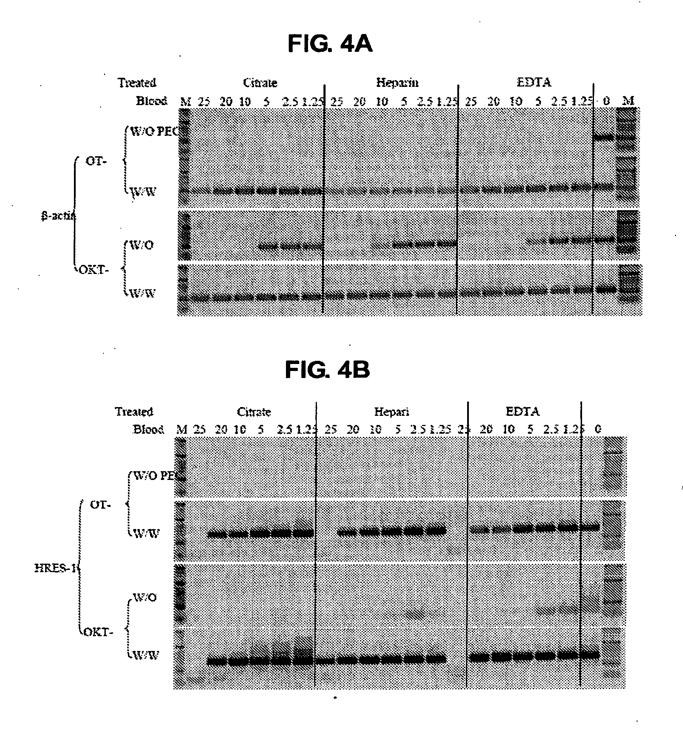

FIG. 4 is a series of agarose gel images depicting amplification efficiency of targets from a blood sample treated with different anticoagulants. FIG. 4A shows amplification of a 300 bp target of the β-actin gene from blood sample treated with citrate, heparin and EDTA using OT or OKT with or without PEC. FIG. 4B shows amplification of a 488 bp target of the HRES-1-c gene from blood sample treated with citrate, heparin and EDTA using OT or OKT with or without PEC. Further details regarding the methodology are provided in Example 4.

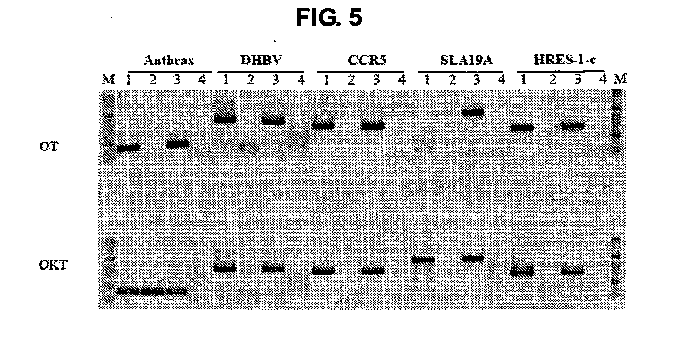

FIG. 5 is a series of agarose gel images depicting direct PCR amplifications of endogenous and exogenous targets from 10% whole blood. A 210 bp of Anthrax Bacillus Bacillus anthracis gene (GC content 33%), 690 bp of duck hepatitis B P gene (DHBV-P, GC content 44%), 630 bp of CCR5 (GC content 47%), 980 bp of SLC19A gene (GC content 74%) and 488 bp of the HRES-1-c gene (GC content 81%) were amplified from a 10% blood sample using OT and OKT in the absence or presence of PEC. In addition, 1.3 M Betaine was included for a comparison. Further details regarding methodology are provided in Example 5.

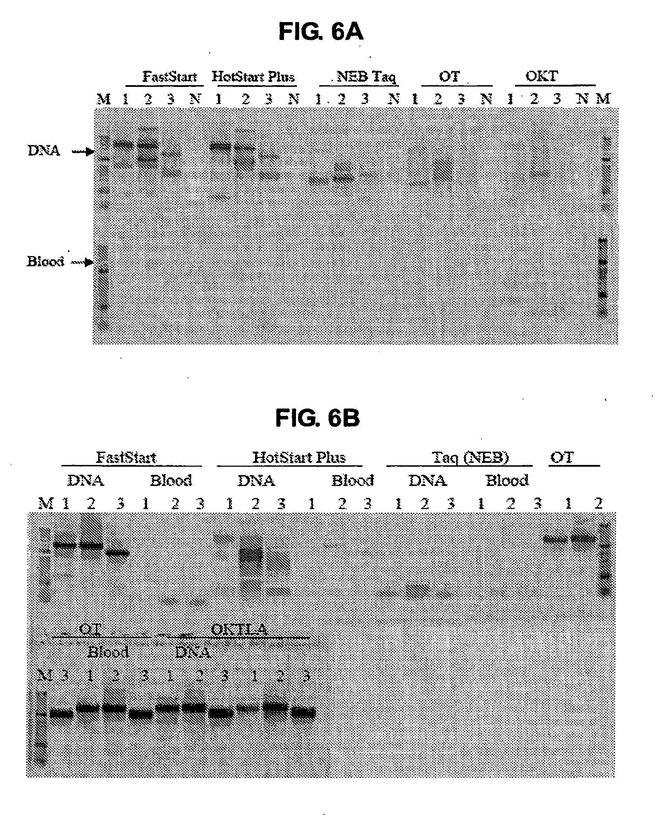

FIG. 6 is a series of agarose gel images comparing OT and OKT vs. commercial enzymes. A1202 bp target of SIM2 gene, 1185 bp target of DIP2A gene and 980 bp target of SLC19A gene were amplified from purified DNA and 5% whole blood in the absence (FIG. 6A) or presence (FIG. 6B) of PCR enhancers. Further details regarding methodology are presented in Example 6.

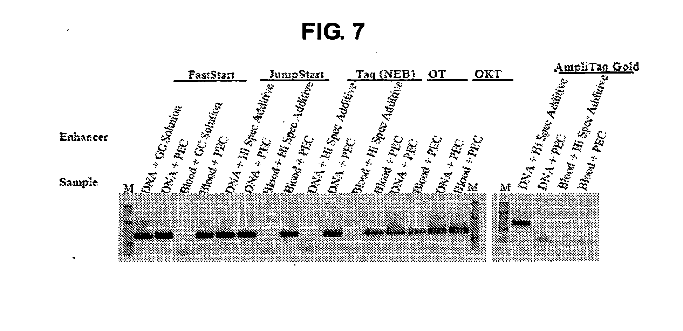

FIG. 7 is a series of agarose gel images depicting a comparison of the effect of PEC vs. commercial PCR enhancers when using various commercial Taqs. A 488 bp target of HRES-1-c was amplified from both purified DNA and blood using wild type Taq, FastStart, JumpStart and AmpliTaq Gold in the presence of commercial enhancers or PEC. Further details regarding methodology are provided in Example 7.

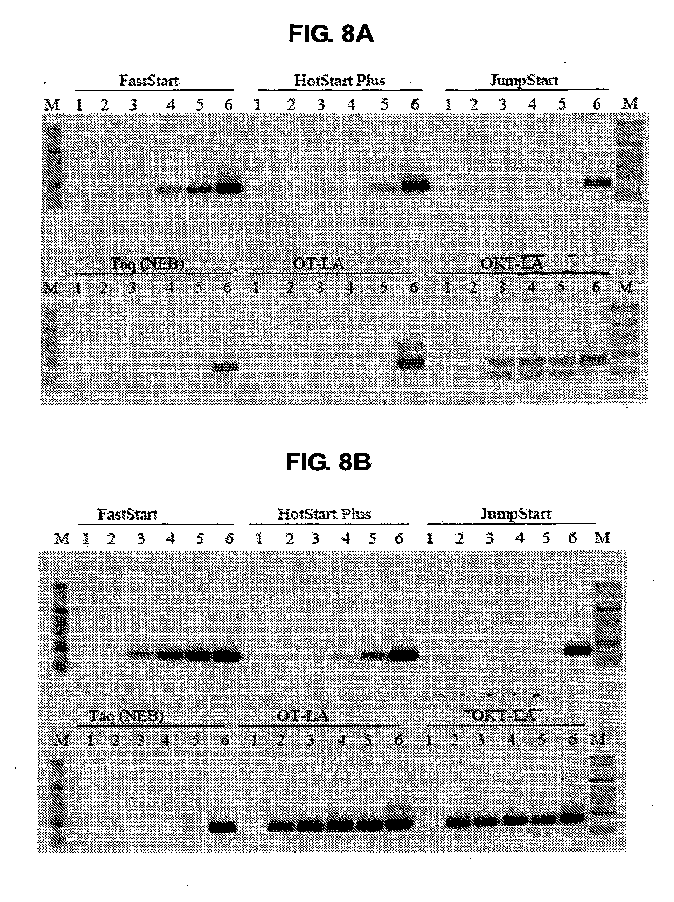

FIG. 8 is a series of agarose gel images depicting a comparison of OT and OKT vs. commercial enzymes on the amplification of a target in soil extract. A 210 bp pag gene target of Anthrax Bacillus was amplified from soil extract in the presence of different PCR enhancers. Further details regarding methodology are presented in Example 8.

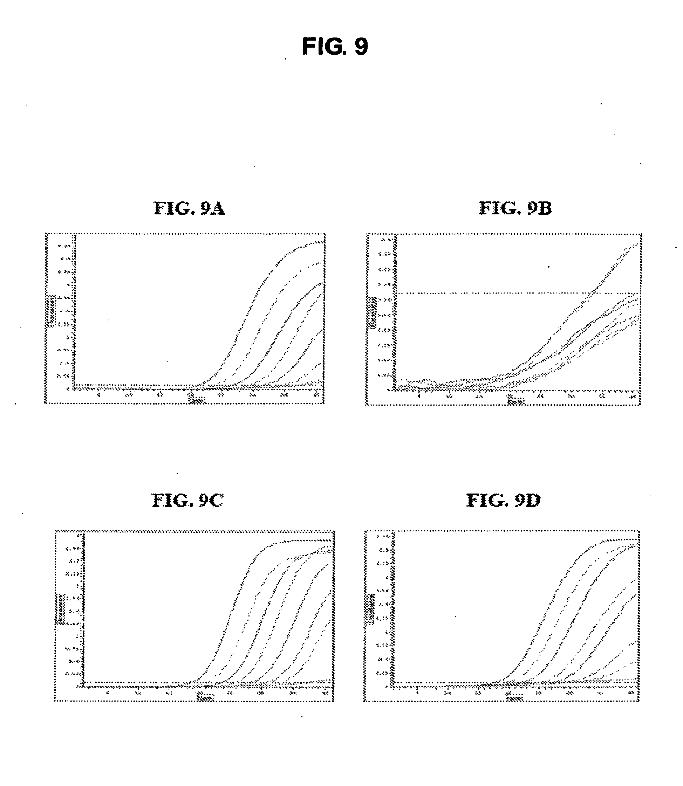

FIG. 9 is a series of line graphs showing fluorescence as a funtion of cycle in real time PCR SYBR green detection assay for whole blood sample and the enhancement effect of PEC thereon. FIG. 9A shows an SYB green amplification curve of a 281 bp mousepox virus gene amplified from 5% whole blood in presence of PEC using OT. FIG. 9B shows an SYB green amplification curve of a 281 bp mousepox virus gene amplified from 5% whole blood in absence of PEC using OT. FIG. 9C shows an SYB green amplification curve of a 281 bp mousepox virus gene amplified from 5% whole blood in presence of PEC using OKT. FIG. 9D shows an SYB green amplification curve of a 281 bp mousepox virus gene amplified from 5% whole blood in absence of PEC using OKT. Further details regarding methodology are presented in Example 9.

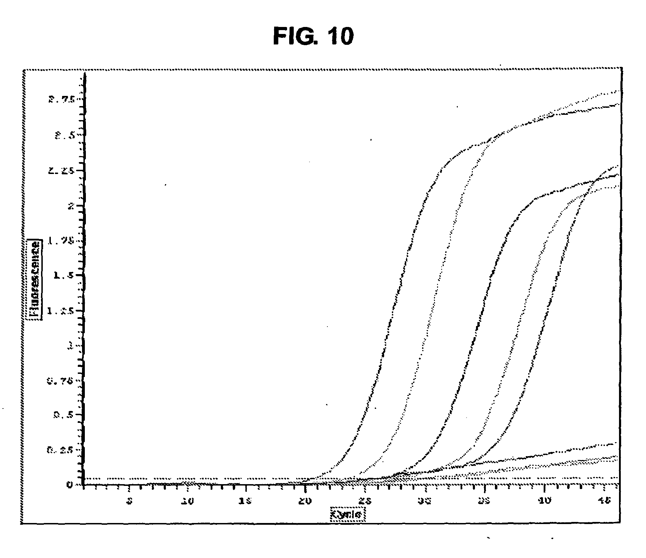

FIG. 10 is a line graph showing fluorescence asa funtion of cycle in real time PCR TaqMan detection for heparin treated plasma sample and the enhancement effect of PEC thereon. A 281 bp mousepox virus gene was amplified from 10% heparin treated plasma in the presence of PEC using OT/OKT mixture. Further details regarding methodology are presented in Example 10.

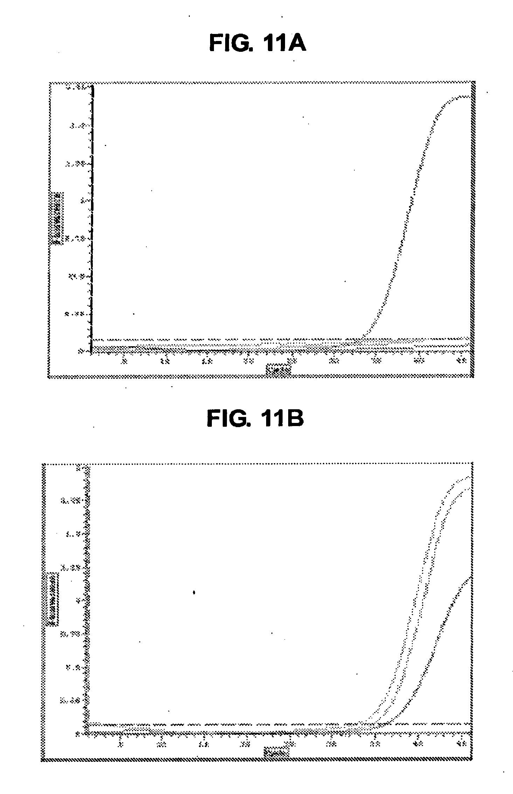

FIG. 11 is a series of line graphs showing fluorescence as a funtion of cycle in real time PCR TaqMan detection for a non-heparin treated plasma and serum sample and the enhancement effect of PEC and PEC-plus (PEC plus a 10u/ml addition of heparin) thereon. FIG. 11A shows a 281 bp mousepox virus gene amplified from a non-heparin treated 10% plasma or 10% serum sample using OT/OKT mixture in presence of PEC. FIG. 11B shows a 281 bp mousepox virus gene amplified from a non-heparin treated 10% plasma or 10% serum sample using OT/OKT mixture in presence of PEC-plus. Further details regarding methodology are presented in Example 11.

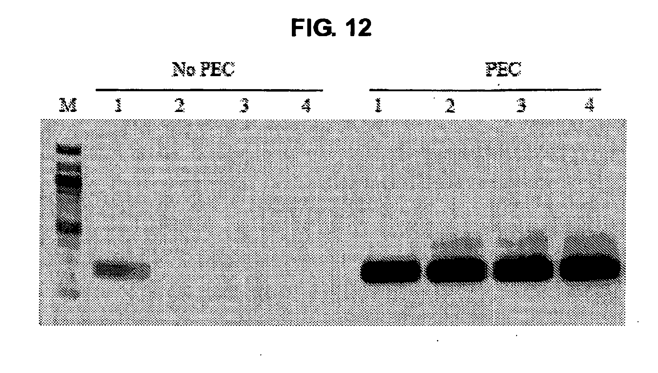

FIG. 12 is a series of agarose gel images depicting enhancement of PECplus on RT-PCR to detect RNA target. A 244 bp influenza virus gene was amplified from 10% citrate-treated plasma, serum and whole blood by one-step RT-PCR using MMLV/OT/OKT mixture in absence or presence of PECplus. Further details regarding methodology are presented in Example 12.

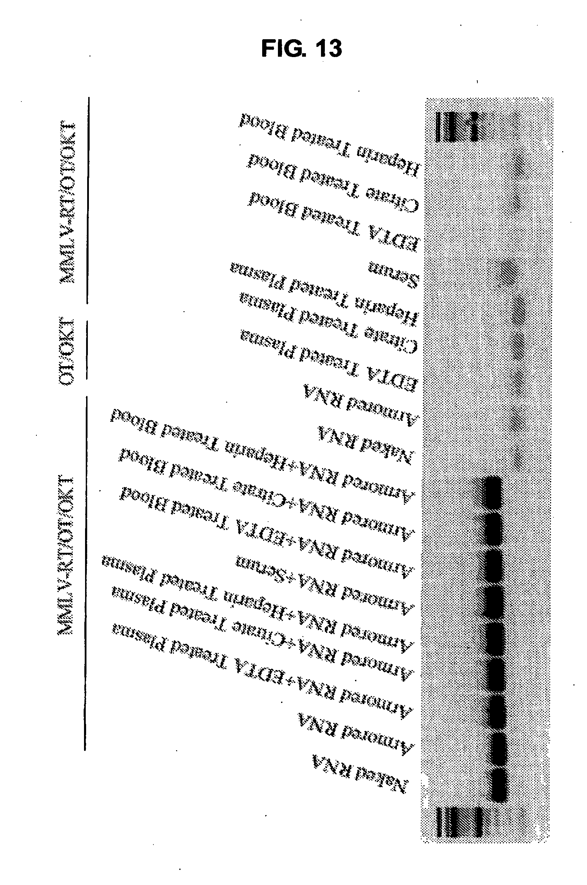

FIG. 13 is an agarose gel image depicting RT-PCR detection of HCV1b from virus-like particles (armored RNA) in crude samples. Lane 1, naked RNA; lane 2, intact armored RNA particles; lane 3-9, intact armored RNA particles mixed with 5µl EDTA treated plasma (lane 3), citrate treated plasma (lane 4), heparin treated plasma (lane 5), serum (lane 6), EDTA treated blood (lane 7), citrate treated blood (lane 8) and heparin treated blood (lane 9), respectively. Lane 10 and 11 were the same as lane 1 and lane 2 but contained no MMLV-H⁻ reverse transcriptase. Lanes 12-18 were the same as lanes 3-9 except that the HCV RNA was omitted. Further details regarding methodology are presented in Example 13.

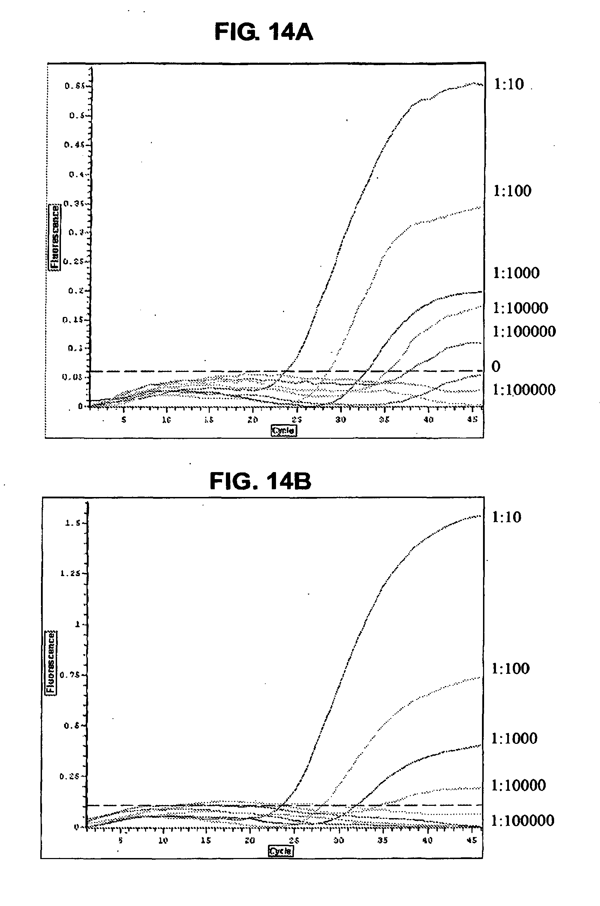

FIG. 14 is series of line graphs showing fluorescence as a function of cycle in real time PCR TaqMan detection of a 244 bp influenza virus RNA target in crude samples containing 5% human serum. FIG. 14A shows fluorescence signal from one-step RT-PCR reactions (25 µl) containing different concentrations of RNA (seven 10-fold dilutions and zero control, as indicated) and constant concentrations of human serum (5%). FIG. 14B shows fluorescence signals from parallel reactions with purified RNA alone, included as a comparison and positive control. Further details regarding methodology are presented in Example 14.

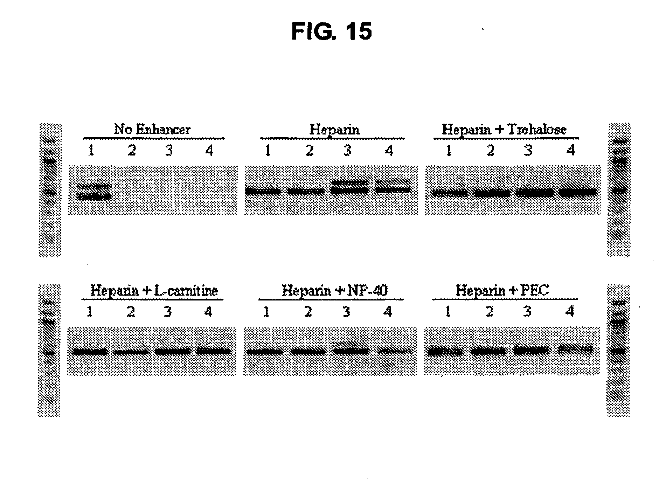

FIG. 15 is a series of agarose gel images showing the role of heparin as a compound of an enhancer for PCR. A 0.5 kb target was amplified from heparin treated plasma (lane 1), citrate treated plasma (lane 2), EDTA treated plasma (lane 3), and serum (lane 4) using Omni Klentaq. The reactions were performed in the presence of heparin alone, heparin combined with D-(+)-trehalose, heparin combined with L-carnitine, heparin combined with NP-40, and heparin combined with PEC (i.e., PEC-Plus). The reactions containing no PCR enhancer were included as a control. The PCR products were resolved in 1.5% agarose gel and stained with ethidium bromide. Further details regarding methodology are presented in Example 15.

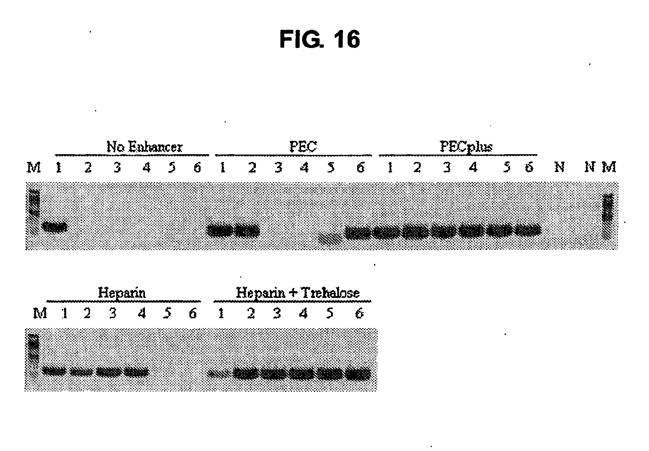

FIG. 16 is a series of agarose gel images showing the role of heparin as a compound of an enhancer for PCR. A 189 bp target of mousepox virus gene was amplified by Omni Taq/Omni Klentaq mix (1:1) from 0.1 ng viral total DNA (lane 1) and crude sample spiked with virus including 10% heparin (lane 2), citrate treated plasma (lane 3), EDTA treated plasma (lane 4), serum (lane 5), and EDTA treated whole blood (lane 6). The reactions were performed in the absence of enhancer, and in the presence of heparin alone, combination of heparin and trehalose, PEC, and PEC-Plus (i.e., PEC plus heparin), as indicated across the top of the gel image. Negative controls contained no virus DNA (N). Further details regarding methodology are presented in Example 15.

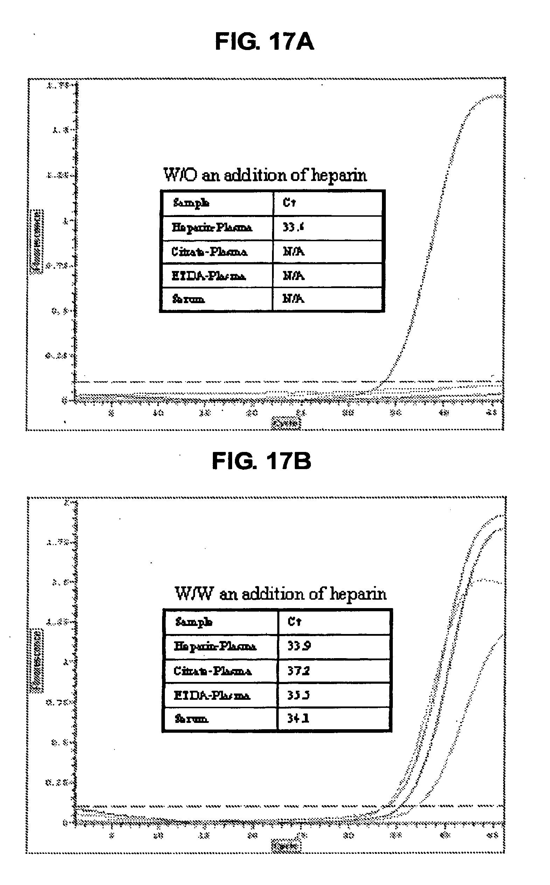

FIG. 17 is a series of line graphs showing the role of heparin in real-time PCR TaqMan assay in crude samples. A 189bp of mousepox virus gene was amplfied from different anticoagulant treated plasma or serum by 2 units of OmniTaq and OmniKlentaq mix with TaqMan assay in the absence (FIG. 17A) or the presence (FIG. 17B) of 10 units heparin / ml plasma or serum. Amplification curves and Ct values are presented.

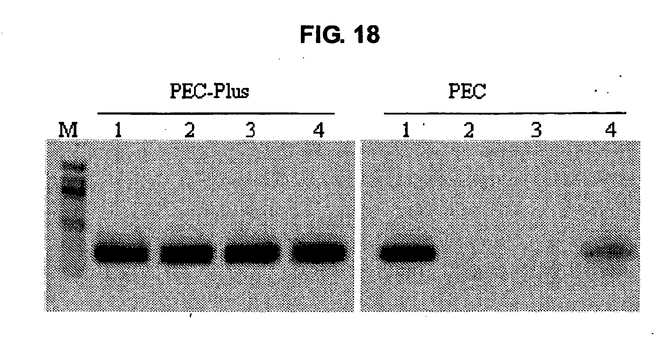

FIG. 18 is a series of agarose gel images showing the role of heparin as a component of an enhancer composition in RT-PCR in crude sample. A 144 BP target of influenza virus gene was amplified by 2 unitof OmniTaq and KlenTaq mix (1:1) and 100 units of MMLV RT from 8% (final concentration in one step RT-PCR) heparin treated plasma (lane 1), citrate treated plasma (lane 2), serum (lane 3) and heparin treated whole blood (lane 4). Reactions were performed in the presence of PEC or PEC-Plus (PEC plus 10 units heparin/ml crude sample), respectively. The PCR products were resolved in 2% agarose gel and stained with ethidium bromide.

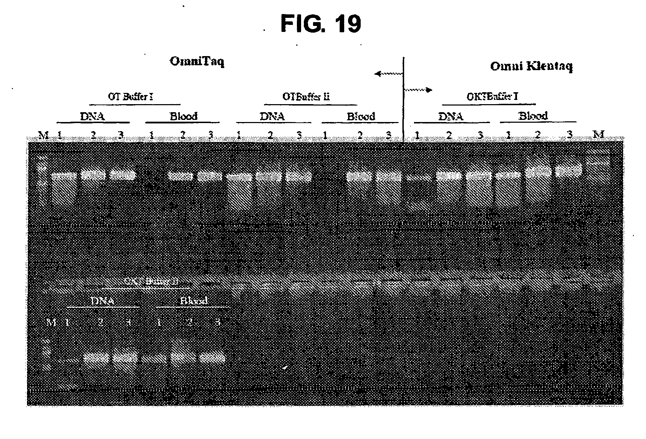

FIG. 19 is a series of agarose gel images showing the performance of enhancer compositions containing NP-40 or Brij-58 in conventional PCR. 690 bp of duck hepatitis B P gene (DHBVP) was amplified from purified DNA and directly from virus-spiked 10% heparin treated whole blood (final concentration in PCR) using OmniTaq (OT) and Omni Klentaq (OKT) in the absence or presence of PEC containing 0.4% NP-40, or PEC containing 0.1 % Brij-58 (polyoxyethylene cetyl ether). Further details regarding methodology are presented in Example 18.

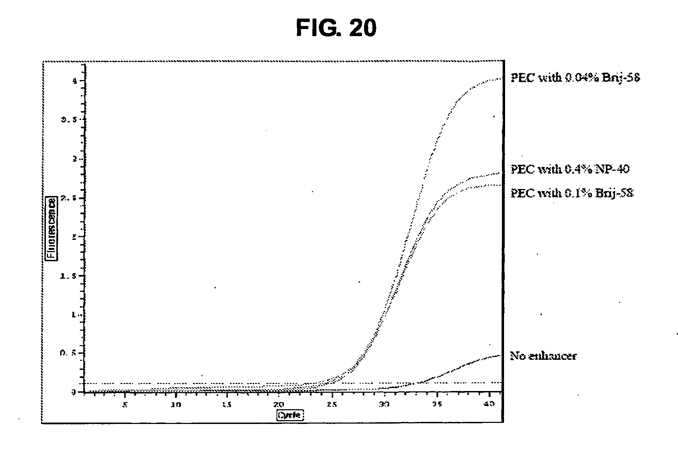

FIG. 20 is a series of line graphs showing performance of enhancer compositions containing NP-40 or Brij-58 in real time PCR with SYBR detection. A 690 bp target of duck hepatitis B P gene (DHBVP) was amplified directly from virus-spiked 5% heparin treated blood (final concentration in PCR) using Omni Klentaq (OKT) in the absence or presence of PEC containing 0.4% NP-40 or PEC containing 0.04%/0.1% Brij-58. Further details regarding methodology are presented in Example 19.

### DETAILED DESCRIPTION OF THE INVENTION

The present invention is based, at least in part, on the discovery of various compositions that can enhance performance of polymerase enzymes, making them more tolerant of various inhibitors. For example, various enhancer compositions described herein allow amplification of nucleic acid molecules in the presence of elevated concentrations of blood, plasma, serum, soil or dye. Various embodiments of the enhancer compositions include the PCR enhancer cocktail (PEC) and the PCR enhancer cocktail plus (PECplus). Also provided herein are methods for amplification (e.g., conventional PCR, real-time PCR, RT-PCR analyses of blood and other samples) utilizing polymerase enzymes (e.g., DNA mutant polymerase enzymes or commercial Taqs) with enhancer compositions described herein.

Briefly, the present invention is directed to an enhancer mixture that can improve performance of polymerase enzymes. Various enhancer compositions described herein can improve the performance of mutant polymerase enzymes, especially under under higher concentrations of PCR inhibitors or against more difficult targets. Further, enhancer compositions described herein can improve performance of wild type polymerase enzymes. For example, most commercial Taq polymerases, except some chemically modified Taqs, such as AmpliTaq Gold, can benefit in their PCR performance from the enhancer mix.

Combining novel Taq mutants and PCR enhancer cocktail (see WO/2008/034110), different GC content targets can be amplified from purified DNA and directly from at least 25% whole blood treated with heparin, citrate and EDTA, plasma and serum without DNA purification prior to PCR reaction. Such an approach not only enhances conventional PCR but also improves real-time PCR performance with, for example, SYBR green fluorescence detection and TaqMan assay. Therefore, the enhancer composition described herein can be combined with wild type and mutants of Taq so as to extremely simplify, speed-up, and lower the cost of important clinical, agricultural, environmental, bio-terrorism related, and forensic tests.

While there presently exists compositions, such as Betaine, Proline, and DMSO, that are used as PCR additives, such compositions are generally restricted to certain functions, such as lowering the melting temperature of DNA or resolving secondary structure of the template, and most work only on substantially purified DNA.

The present invention, however, provides an additive that can work on lower and higher GC content targets, purified DNA, and crude samples that may contain PCR inhibitors. As shown herein, non-GC-rich targets showed a more trehalose dependent pattern while GC-rich targets showed a more L-carnitine dependent pattern (see e.g., FIG. 1A, 1B). In one embodiment, the enhancer composition contains an optimized concentration of components including 0.6 M D-(+)-trehalose, 0.48 M L-carnitine, and 0.8% NP-40, which can amplify both lower GC and higher GC content targets. Such an enhancer can outperform the individual components of the cocktail (see e.g. FIG. 1 C) and can amplify targets both from purified DNA and blood.

Amplification of a higher GC rich target is difficult and usually cannot yield a specific product. As shown herein, various embodiments of the enhancer composition (e.g., PEC or PECplus) can increase the specificity of PCR reaction (see e.g. FIG. 1C, 2A).

As shown herein, OKT has a blood-resistant feature and can work on an "easy" target with lower concentrations of blood or soil without additives (see e.g., FIG. 4A, FIG. 8). Among the benefits of various aspects of the invention is combining a blood-resistant mutant Taq with an enhancer such as PEC so as to accommodate a large quantity of blood, plasma, serum or soil in the amplification reactions, such as conventional PCR or real-time PCR, directly amplifying no-GC rich or GC-rich targets without DNA purification prior to PCR (see e.g. FIG. 1-11). Also demonstrated herein, use of enhancer compositions with crude samples does not compromise the sensitivity of the reaction (see e.g. FIG. 2B).

Citrate, Heparin and EDTA are major anticoagulants applied in clinical diagnosis. As shown herein, OT and OKT can amplify non-GC rich or GC-rich targets from different concentrations of blood samples treated with different anticoagulant in the presence of an enhancer composition, such as PEC (see e.g. FIG. 4A, B). Such a method can provide for the broad application of enhancer compositions, such as PEC, in PCR reactions for clinical diagnosis.

Furthermore, various commercial polymerases (e.g., Taq polymerase) fail to work in blood-containing PCR reactions due to PCR inhibitors present in the sample. But with an enhancer composition described herein, a commercial polymerase can directly amplify a target from a blood sample (see e.g. FIG. 7). This, enhancer compositions described herein have broad application in PCR for wild type and mutant polymerases.

Enhancer compositions, such as PEC and PEC-Plus, can not only improve the performance of conventional PCR, but can also improve RT-PCR and real-time PCR (including, for example, SYBR green assay or TaqMan detection of targets) for differently treated whole blood, plasma and serum (see e.g., FIG. 9,10). Such results demonstrate application of enhancer compositions described herein in clinical PCR diagnositics.

Thus, combining blood-resistant Taq-mutants or other commercial Taq DNA polymerases or RNA reverse transcriptase with enhancer compositions, such as PEC or PECplus, provides a valuable tool for an improved, low-cost, fast, and sensitive PCR detection of genes generally, including clinical applications in specimens with or without crude blood.

### ENHANCER COMPOSITION

An enhancer composition of the present invention comprises one or more of trehalose, carnitine, a nonionic detergent, and heparin.

In some embodiments, an enhancer composition of the present invention comprises trehalose, carnitine, and a nonionic detergent (e.g., PEC). As demonstrated herein, an enhancer composition comprising trehalose, carnitine, and a nonionic detergent (e.g., NP-40) has a synergistic effect on the efficiency of an amplification reaction as compared to each component individually.

In some embodiments, an enhancer composition of the present invention comprises heparin alone or in combination with one of more of trehalose, carnitine, and a nonionic detergent. For example, an enhancer composition can comprise trehalose, carnitine, a nonionic detergent, and heparin

(e.g., PEC Plus). As demonstrated herein, an enhancer composition comprising heparin (either alone or in combination with one or more of trehalose, carnitine, and a nonionic detergent) can overcome inhibition of an amplification reaction in crude samples, such as plasma or serum. Preferably, an enhancer composition for use in crude sample (e.g., plasma or serum containing) comprises heparin.

In some embodiments, an enhancer composition comprises 0.1 to 0.8 M trehalose, 0.1 M to 1.5 M L-carnitine, and 0.1 to 8% nonionic detergent, such as NP-40. In one embodiment, the enhancer composition contains components including about 0.6 M D-(+)-trehalose, about 0.48 M L-carnitine, and about 0.8% NP-40 (PEC). In another embodiment, the enhancer composition contains components including about 0.6 M D-(+)-trehalose, about 0.48 M L-carnitine, about 0.8% NP-40, and about 10 units/mL heparin (PEC-Plus). Further concentration ranges, and combinations thereof, are discussed further below.

The formulation of an enhancer composition can be tailored to the GC content of the target nucleic acid. For example, for lower GC targets, an enhancer composition can comprise about 0.64 M trehalose, about 0.12 M L-carnitine, and about 0.8% NP-40. As another example, for average GC targets, an enhancer composition can comprise about 0.64 M trehalose, about 0.24 M L-carnitine, and about 0.8% NP-40. As another example, for high GC targets, an enhancer composition can comprise about 0.48 to about 0.64 M trehalose, about 0.48 to about 1.44 M L-carnitine, and about 0.8% NP-40.

Various embodiments can include one or more additional components selected from betaine, DTT, dimethyl sulfoxide (DMSO), BSA, glycerol, formamide, ammonium ions (e.g., (NH)₄SO₄), polyethylene glycol, and tetramethyl ammonium chloride.

Component concentrations herein are expressed in terms of concentration in the final amplification reaction mixture volume, unless indicated otherwise. In some embodiments, an enhancer composition is in concentrated form such that when added to a reaction mixture volume, the concentration of components recited herein results. For example, an enhancer composition can be 2X (two-fold concentrated), such that the component concentration in the concentrated enhancer composition is two-times higher than the actual final concentration of the enhancer components in a reaction mixture.

### TREHALOSE

Trehalose (2-(hydroxymethyl)-6-[3,4,5-trihydroxy-6-(hydroxymethyl) tetrahydropyran-2-yl] oxy- tetrahydropyran-3,4,5-triol), or mycose or tremalose, is a natural α-linked disaccharide formed of an α, α-1, 1-glucoside bond between two α-glucose units. trehalose can be used as an anhydride or dihydrate form. trehalose can be obtained from a variety of commercial sources (e.g., Sigma-Aldrich, St. Louis, MO).

An enhancer composition can comprise an amount of trehalose effective to increase the efficiency of an amplification reaction. An enhancer composition can comprise, for example, 0.1 M to 1.0 M trehalose; 0.2 M to 1.0 M trehalose; 0.3 M to 0.9 M trehalose; 0.4 to 0.8 M trehalose; or 0.5 M to 0.7 M trehalose. Preferably, an enhancer composition comprises 0.3 to 0.8 M trehalose. An enhancer composition can comprise, for example, 0.1 M, 0.2 M, 0.3 M, 0.4 M, 0.5 M, 0.6 M, 0.7 M, 0.8 M, 0.9 M, or 1.0 M, or more, of trehalose. Preferably, an enhancer composition comprises about 0.6 M trehalose. Concentrations discussed above are expressed in terms of concentration in a final amplification reaction mixture volume. Calculation of a corresponding concentration in an isolated (and concentrated) enhancer composition is within the skill of the art. The above concentrations of trehalose can be combined with any of the concentrations of carnitine, a nonionic detergent, or heparin discussed herein.

### CARNITINE

Carnitine (3-Hydroxy-4-trimethylammonio-butanoate) is a quaternary ammonium compound biosynthesized from the amino acids lysine and methionine. The biologically active form is L-carnitine, while its enantiomer, D-carnitine, is biologically inactive. Either L-carnitine or D-carnitine, or both, can be included in the enhancer composition. Preferably, the enhancer composition contains L-carnitine. carnitine can be obtained from a variety of commercial sources (e.g., Sigma-Aldrich, St. Louis, MO).

An enhancer composition can comprise an amount of carnitine effective to increase the efficiency of an amplification reaction. In some embodiments, an enhancer composition can comprise, for example, 0.1 M to 1.5 M carnitine; 0.1 M to 1.4 M carnitine; 0.1 M to 1.3 M carnitine; 0.1 M to 1.2 M carnitine; 0.1 M to 1.1 M carnitine; 0.1 M to 1.0 M carnitine; or 0.1 M to 1.0 M carnitine. In other embodiments, an enhancer composition can comprise, for example, 0.1 M to 1.0 M carnitine; 0.2 M to 0.8 M carnitine; 0.3 M to 0.7 M carnitine; or 0.4 M to 0.6 M carnitine. Preferably, an enhancer composition comprises 0.12 M to 0.72 M carnitine. An enhancer composition can comprise, for example, 0.1 M, 0.2 M, 0.3 M, 0.4 M, 0.5 M, 0.6 M, 0.7 M, 0.8 M, 0.9 M, 1.0 M; 1.1 M; 1.2 M; 1.3 M; 1.4 M; or 1.5 M, or more, of carnitine. Preferably, an enhancer composition comprises about 0.5 M carnitine, more preferably 0.48 M carnitine. Concentrations discussed above are expressed in terms of concentration in a final amplification reaction mixture volume. Calculation of a corresponding concentration in an isolated (and concentrated) enhancer composition is within the skill of the art. The above concentrations of carnitine can be combined with any of the concentrations of trehalose, a nonionic detergent, or heparin discussed herein.

### DETERGENT

An enhancer composition can comprise an amount of a biological detergent, preferably a nonionic detergent, more preferably a nonionic detergent, non-denaturing detergent, effective to increase the efficiency of an amplification reaction. Preferably, a nonionic, non-denaturing detergent to be included in the enhancer composition is not powerful enough to break the nuclear membrane, but can break the cytoplasmic membrane. Examples of nonionic detergents include, but are not limited to, those listed in Zoller, ed. 2008 Handbook of Detergents, CRC, ISBN-10: 142009162X.

A nonionic detergent can be selected from ethoxylated fatty alcohol ethers and lauryl ethers, ethoxylated alkyl phenols, octylphenoxy polyethoxy ethanol compounds, modified oxyethylated and/or oxypropylated straight-chain alcohols, polyethylene glycol monooleate compounds, polysorbate compounds, or phenolic fatty alcohol ethers. One example of a nonionic, non-denaturing detergent for inclusion in an enhancer composition is Tergitol-type NP-40, or NP-40, which is nonyl phenoxylpolyethoxylethanol. Another example of a nonionic, non-denaturing detergent for inclusion in an enhancer composition is Nonidet P-40 (octyl phenoxylpolyethoxylethanol). Another example of a nonionic, non-denaturing detergent for inclusion in an enhancer composition is Igepal CA-630. Another example of a nonionic, non-denaturing detergent for inclusion in an enhancer composition is a polyoxyethylene cetyl ether, such as Brij-58. Other examples of a nonionic detergent for inclusion in an enhancer composition are Tween®-20, NP-40, and Triton® X-100. Suitable detergents can be obtained from a variety of commercial sources (e.g., Sigma-Aldrich, St. Louis, MO).

An enhancer composition can comprise an amount of biological detergent effective to increase the efficiency of an amplification reaction. An enhancer composition can comprise, for example, at least about 0.01 % non-ionic detergent. An enhancer composition can comprise, for example, up to about 4% non-ionic detergent. An enhancer composition can comprise, for example, about 0.01%; about 0.02%; about 0.03%; about 0.04%; about 0.05%; about 0.06%; about 0.07%; about 0.08%; about 0.09%; about 0.1 %; about 0.11 %; about 0.12%; about 0.13%; about 0.14%; about 0.15%; about 0.16%; about 0.17%; about 0.18%; about 0.19%; or about 0.2%; about 0.3%; about 0.4%; about 0.5%; about 0.6%; about 0.7%; about 0.8%; about 0.9%; about 1.0%; about 1.1 %; about 1.2%; about 1.3%; about 1.4%; about 1.5%; about 1.6%; about 1.7%; about 1.8%; about 1.9%; about 2%; about 2.1 %; about 2.2%; about 2.3%; about 2.4%; about 2.5%; about 2.6%; about 2.7%; about 2.8%; about 2.9%; about 3%; about 3.1%; about 3.2%; about 3.3%; about 3.4%; about 3.5%; about 3.6%; about 3.7%; about 3.8%; about 3.9%; about 4%; about 4.1%; about 4.2%; about 4.3%; about 4.4%; about 4.5%; about 4.6%; about 4.7%; about 4.8%; about 4.9%; about 5%; about 5.1 %; about 5.2%; about 5.3%; about 5.4%; about 5.5%; about 5.6%; about 5.7%; about 5.8%; about 5.9%; about 6%; about 6.1 %; about 6.2%; about 6.3%; about 6.4%; about 6.5%; about 6.6%; about 6.7%; about 6.8%; about 6.9%; about 7%; about 7.1%; about 7.2%; about 7.3%; about 7.4%; about 7.5%; about 7.6%; about 7.7%; about 7.8%; about 7.9%; about 8%, or more of non-ionic detergent. Preferably, an enhancer composition comprises about 0.8% non-ionic detergent. Concentrations discussed above are expressed in terms of concentration in a final amplification reaction mixture volume. Calculation of a corresponding concentration in an isolated (and concentrated) enhancer composition is within the skill of the art.

An enhancer composition can comprise, for example, about 0.01% to about 8% non-ionic detergent. An enhancer composition can comprise, for example, 0.02% to 7.5% non-ionic detergent; 0.03% to 7% non-ionic detergent; 0.04% to 6.5% non-ionic detergent; 0.05% to 6% non-ionic detergent; 0.06% to 5.5% non-ionic detergent; 0.07% to 5% non-ionic detergent; 0.08% to 4.5% non-ionic detergent; 0.09% to 4% non-ionic detergent; 0.1 % to 3.5% non-ionic detergent; 0.1% to 3% non-ionic detergent; 0.1% to 2.5% non-ionic detergent; 0.1% to 2% non-ionic detergent; 0.1% to 1.5% non-ionic detergent; 0.2% to 1.4% non-ionic detergent; 0.3% to 1.3% non-ionic detergent; 0.4% to 1.2% non-ionic detergent; 0.5% to 1.1 % non-ionic detergent; 0.6% to 1.0% non-ionic detergent; 0.7% to 0.9% non-ionic detergent. An enhancer composition can comprise, for example, 0.1 % to 1.1%; 0.2% to 1.0%; 0.3% to 0.9%; or 0.4% to 0.8%. Preferably, an enhancer composition comprises 0.4% to 0.8% non-ionic detergent. Concentrations discussed above are expressed in terms of concentration in a final amplification reaction mixture volume. Calculation of a corresponding concentration in an isolated (and concentrated) enhancer composition is within the skill of the art.

Any of the specific concentrations of non-ionic detergent discussed above can refer to, for example, Brij-58, NP-40, Nonidet P-40, Igepal CA-630, Tween®-20, NP-40, or Triton® X-100.

In some embodiments, the nonionic detergent in the enhancer composition is a nonyl phenoxylpolyethoxylethanol, such as NP-40, at a concentration of about 0.1% to about 1.1%; about 0.2% to about 1.0%; about 0.3% to about 0.9%; or about 0.4% to about 0.8%. Preferably, the nonionic detergent in the enhancer composition is a nonyl phenoxylpolyethoxylethanol, such as NP-40, at a concentration of about 0.4% to about 0.8%. Concentrations discussed above are expressed in terms of concentration in a final amplification reaction mixture volume. Calculation of a corresponding concentration in an isolated (and concentrated) enhancer composition is within the skill of the art.

In some embodiments, the nonionic detergent in the enhancer composition is a polyoxyethylene cetyl ether, such as Brij-58, at a concentration of at least about 0.01 %. In some embodiments, the nonionic detergent in the enhancer composition is a polyoxyethylene cetyl ether, such as Brij-58, at a concentration of up to about 4%. For exampe, the nonionic detergent in the enhancer composition is a polyoxyethylene cetyl ether, such as Brij-58, at a concentration of 0.01 % to 4.0%; 0.02% to 1.0%; 0.03% to 0.5%; or 0.04% to 0.2% per amplification reaction mixture volume. As another example, the enhancer composition can contain a polyoxyethylene cetyl ether, such as Brij-58, at a concentration of about .01%; about .02%; about .03%; about .04%; about 0.05%; about 0.06%; about 0.07%; about 0.08%; about 0.09%; about 0.1%; about 0.11%; about 0.12%; about 0.13%; about 0.14%; about 0.15%; about 0.16%; about 0.17%; about 0.18%; about 0.19%; or about 0.2%. Concentrations discussed above are expressed in terms of concentration in a final amplification reaction mixture volume. Calculation of a corresponding concentration in an isolated (and concentrated) enhancer composition is within the skill of the art.

### HEPARIN

As shown herein, heparin alone or in combination with other components of an enhancer composition not only helps PCR in whole blood samples but also benefits qPCR (real-time PCR) and RT-PCR in all crude blood-derived samples treated with commonly used anticoagulants. This is the first report demonstrating that heparin can be used as a PCR enhancer and facilitator, by virtue of relieving the inhibitory effect in all kind of blood or crude samples with common anticoagulants.

In some embodiments, an enhancer composition of the present invention comprises heparin. For example, the enhancer composition can consist essentially of heparin. As another example, the enhancer composition can comprise heparin and one or more, two or more, or all of trehalose, carnitine, and a non-ionic detergent. For example, the enhancer composition can comprise heparin, trehalose, carnitine, and a non-ionic detergent. An enhancer composition comprising heparin can be used to increase the efficiency of a variety of amplification reactions including, but not limited to PCR, RT-PCR, real time PCR, and real time RT-PCR, expecially in crude samples.

Heparin is a highly-sulfated glycosaminoglycan, which acts as an anticoagulant and has a high negative charge density. Native heparin polymer has a molecular weight of 3 kDa to 50 kDa, while most commercial heparin preparations have a molecular weight of 12 kDa to 15 kDa. One unit of heparin is an amount approximately equivalent to 0.002 mg of pure heparin, which is understood as the quantity required to keep 1 mL of cat's blood fluid for 24 hours at 0°. Heparin can be obtained from a variety of commercial sources.

Heparin alone can be inhibitory to amplification of purified DNA or RNA substrate. While an enhancer composition comprising heparin and one or more of trehalose, carnitine, and a detergent can be used to enhance amplification of purified DNA, preferably, an enhancer composition for use with purified DNA or RNA substrate contains relatively low levels of heparin, substantially no heparin, or, more preferably, no heparin.

An enhancer composition can comprise an amount of heparin effective to increase the efficiency of an amplification reaction. Inclusion of heparin in an enhancer composition can be desirable where the amplification reaction contains blood or a blood fraction, such as plasma or serum. Citrate, EDTA, and heparin are common anticoagulants used for blood treatment in diagnostics. As shown herein, heparin alone or in combination with other enhancer compounds (e.g., carnitine, trehalose, or a detergent) can enhance PCR amplification in blood or crude blood samples treated with common anticoagulants (see Example 15; FIG. 15; FIG. 16).

Crude blood samples such as plasma or serum contain less heme/hemoglobin than whole blood and, thus, would be expected to have less inhibitory effect on PCR performance than whole blood. Surprisingly, it was observed that OmniTaq (FL-22) and Omni Klentaq (Klentaq-10) could amplify targets from whole blood treated with citrate, EDTA, or heparin; while, with targets from plasma, OmniTaq and Omni Klentaq were only active in heparin treated plasma (see e.g., FIG. 15, No Enhancer, lane 1), but not in the citrate treated plasma and EDTA treated plasma (see e.g., FIG. 15, No Enhancer, lanes 2 and 3, respectively). Similarly, it was observed that OmniTaq (FL-22) and Omni Klentaq (Klentaq-10) could not amplify targets from plasma treated with PEC in absence of heparin (see e.g., FIG. 16, PEC, lanes 3-4) but could amplify targets from plasma treated with PEC in the presence of heparin (see e.g., FIG. 16, PEC, lane 2; PECplus, lanes 2-4). Furthermore, it was observed that OmniTaq (FL-22) and Omni Klentaq (Klentaq-10) along with MMLV reverse transcriptase in an RT-PCR reaction could not amplify targets from plasma or serum treated with PEC in absence of heparin (see e.g., FIG. 18, PEC, lanes 2-3) but could amplify targets from plasma and serum treated with PEC in the presence of heparin (see e.g., FIG. 18, PEC-Plus, lanes 1-3 and PEC, lane 1).

As shown herein, heparin can bind potent PCR inhibitors, such as IgG (immunoglobulins) and lactoferrin (see e.g., Example 15). Also shown herein, heparin alone can relieve the inhibitory action at least in citrate and EDTA treated plasma samples (see e.g., Example 15; FIG. 16, Heparin, lanes 3 and 4, respectively). And adding heparin to PEC (i.e., PEC-Plus) can allow efficient amplification from purified nucleic acid targets as well as crude plasma, serum, or blood specimens by OmniTaq and Omni Klentaq (see e.g., Example 15; FIG. 16, PECplus, lanes 1-6). Heparin can also increase efficiency of reverse transcriptase containing amplification reactions, such as RT-PCR, especially in crude samples (see e.g., Example 17; FIG. 18, PEC-Plus, lanes 1-4 and PEC, lanes 1 and 4).

While these findings were demonstrated for OmniTaq and Omni Klentaq, heparin can have similar effects on other DNA polymerases, such as Taq. While other DNA polymerases such as wild type Taq are more sensitive to PCR inhibitors, they can still tolerate some concetrations of them in the presence of an enhancer composition, such as PEC, and more so with a heparin-containing enhancer composition, such as PEC-Plus.

Thus is demonstrated that heparin plays a role in overcoming inhibition of PCR in crude blood samples (including PCR, RT-PCR, real time PCR, and real time RT-PCR), such as those containing plasma or serum.

An enhancer composition can comprise an amount of heparin effective to increase the efficiency of an amplification reaction. An enhancer composition can comprise, for example, 2 units to 50 units of heparin per mL of crude blood sample (e.g., plasma or serum). For example, an enhancer composition can comprise 5 units to 40 units, 5 units to 35 units, 5 units to 30 units, 5 units to 25 units, 5 units to 20 units, 5 units to 15 units, 6 units to 14 units, 7 units to 13 units, 8 units to 12 units, or 9 units to 11 units of heparin per mL of crude blood sample (e.g., plasma or serum). Preferably, an enhancer composition comprises about 10 units of heparin per mL of crude blood sample (e.g., plasma or serum). Concentrations discussed above are expressed in terms of concentration per crude blood sample volume (i.e., not final reaction mixture volume). Calculation of a corresponding concentration in an isolated (and concentrated) enhancer composition is within the skill of the art.

### ADDITION OF ENHANCER

The enhancer composition can be introduced into an amplification reaction before, during, or after introduction of a template molecule. The enhancer composition can be introduced into an amplification reaction before, during, or after introduction of primers. The enhancer composition can be introduced into an amplification reaction before, during, or after introduction of a polymerase enzyme. The enhancer composition can be introduced into an amplification reaction before, during, or after introduction of deoxynucleoside triphosphates. The enhancer composition can be introduced into an amplification reaction before, during, or after introduction of a buffer solution. The enhancer composition can be introduced into an amplification reaction before, during, or after introduction of divalent cations, such as magnesium or manganese ions. The enhancer composition can be introduced into an amplification reaction before, during, or after introduction of monovalent cations, such as potassium ions.

### AMPLIFICATION REACTIONS

An enhancer composition described herein can be used in conjunction with a variety of gene amplification processes well known in the art (see e.g., Dorak (2006) Real-Time PCR, Taylor & Francis, ISBN 041537734X; Bustin, ed. (2004) A-Z of Quantitative PCR, International University Line, ISBN 0963681788). Examples of nucleic acid amplification processes for use with the enhancer composition of the present invention include, but are not limited to, Allele-specific PCR; Assembly PCR or Polymerase Cycling Assembly; Asymmetric PCR; Linear-After-The-Exponential-PCR; Helicase-dependent amplification; Hot-start PCR; Intersequence-specific PCR; Inverse PCR; Ligation-mediated PCR; Methylation-specific PCR; Miniprimer PCR; Multiplex Ligation-dependent Probe Amplification; Multiplex-PCR; Nested PCR; Overlap-extension PCR; Quantitative PCR; Quantitative End-Point PCR; Quantitative Real-Time PCR; RT-PCR (Reverse Transcription PCR); Solid Phase PCR; Thermal asymmetric interlaced PCR; Touchdown PCR; PAN-AC; Universal Fast Walking; Long PCR; Rapid Amplified Polymorphic DNA Analysis; Rapid Amplification of cDNA Ends (RACE); Differential Display PCR; In situ PCR; High-Fidelity PCR; PCR and DNA Sequencing (cycle sequencing).

For example, PCR is commonly carried out in a reaction volume of about 10-200 µl in small reaction tubes (about 0.2-0.5 ml volumes) in a thermal cycler. An enhancer composition can be combined with the PCR reaction mixture at any step including, initialization, denaturation, annealing, extension/elongation, final elongation, or hold. Preferably, the enhancer composition is combined with a PCR reaction mixture so as to be present during extension and elongation.

The use of an enhancer composition described herein generally does not require any, or substantial, changes in a typical protocol, other than, for example, combining the enhancer composition and the reaction mixture. Thus, compositions and methods described herein can be applied to improve the nucleic acid detection in any standard PCR protocol with, for example, crude samples.

Provided herein is a method for improving performance in PCR amplification of a heterogeneous mixture of nucleic acid templates. The heterogeneous mixture can include endogenous DNA or RNA (e.g., present in blood cells) or exogenous DNA or RNA (e.g., from a pathogen). Such a method generally includes forming an assay mixture from nucleic acid templates of varying G+C content; an enhancer composition described herein; and at least one polymerase. The assay mixture is then reacted so as to amplify the nucleic acid templates.

Also provided is a method for improving performance in real-time reverse transcriptase (RT) PCR amplification of an RNA target. An enhancer composition described herein can be used in a variety of RT-PCR protocols known to the art (see e.g., King and O'Connel (2002) RT-PCR Protocols, 1st Ed., Human Press, ISBN-10 0896038750). It is noted that reverse transcriptase (RT) PCR is not to be confused with real-time polymerase chain reaction (Q-PCR), which is sometimes (incorrectly) abbreviated as RT-PCR in the art. In RT-PCR, an RNA strand is first reverse transcribed into its DNA complement (complementary DNA, or cDNA) using the enzyme reverse transcriptase, and the resulting cDNA is amplified using traditional PCR. Applications of RT-PCR include, but are not limited to, detection of RNA virus pathogens; analysis of mRNA expression patterns of certain genes related to various deseases; semiquantitative determination of abundance of specific different RNA molecules within a cell or tissue as a measure of gene expression; and cloning of eukaryotic genes in prokaryotes. A target RNA in an RT-PCR reaction including an enhancer composition can be, for example, an RNA of viral origin or an RNA of cellular origin. An RT-PCR reaction can comprise at least one polymerase, or preferably a mixture of polymerases as discussed above, and at least one reverse transcriptase. Selection of appropriate polymerases and reverse transcriptases is within the skill of the art. An enhancer composition can be combined with the RT-PCR reaction mixture at any step.

Also provided is a method for improving performance in dye-containing amplification reactions through addition of an enhancer composition described herein. In some embodiments, an enhancer composition is added to a real-time PCR (qPCR) reaction mixture to overcome inhibitory effects of dyes (e.g., fluorescent dyes) used in qPCR. Dyes for use in the methods described herein include, but are not limited to, SYBR Green (Molecular Probes, Eugene, Oregon), LC Green (Idaho Technology, Salt Lake City, Utah), PicoGreen (Molecular Probes, Eugene, Oregon,), TOTO (Molecular Probes, Eugene, Oregon), YOYO (Molecular Probes, Eugene, Oregon) and SYTO9 (Molecular Probes, Eugene, Oregon). Dye-resistance can be readily determined by assays known in the art. Various embodiments of the enhancer compositions described herein can increase polymerase tolerance against increased concentrations of dyes. Such increased concentrations include, but are not limited to, up to about 0.5X, 1X, 1.5X, 2X, 2.5X, 3X, 3.5X, 4 X, 4.5X, 5X, 5.5X, 6X, 6.5X, 7X, 7.5X, 8X, 8.5X, 9X, 9.5X, 10X, 15X, 20X, 25X, 30X, 35X, 40X, 45X, 50X, 55X, 60X, 65X, 70X, 80X, 90X, 100X, 150X, 200X, 250X, or even higher over the dye concentration conventionally used in the assay. As an example, X can be the standard manufacturers unit for dye concentration provided in a commercial product (e.g., SYBR Green, Molecular Probes, Eugene, Oregon). For example, where the dye is SYBR Green, 1X can correspond to a concentration of about 1µg/ml.

Also provided is a method for improving performance in blood-containing amplification reactions (e.g., PCR, qPCR, RT-PCR, real-time RT-PCR) through addition of an enhancer composition described herein. An enhancer composition can improve performance in the presence of whole blood or blood components. Blood components include, but are not limited to, red blood cells, white blood cells (e.g., leukocytes or platelets, i.e., thrombocytes), plasma, serum, hemoglobin, water, proteins, glucose, amino acids, fatty acids, mineral ions, hormones, carbon dioxide, urea, and lactic acid. An enhancer composition described herein can be used in a PCR to aid amplification of a nucleic acid target in the presence of one or more such blood components.

Blood plasma is generally understood as a liquid suspension in which blood cells are circulated. Thus, blood plasma can include one or more of water, proteins, glucose, amino acids, fatty acids, mineral ions, hormones, carbon dioxide, urea, lactic acid, platelets (i.e., thrombocytes), and blood cells. In a human subject, blood plasma represents about 55% of whole blood, or about 2.7 to 3 liters in an average human subject. Blood plasma contains about 92% water, 8% blood plasma proteins, and trace amounts of other materials. Blood plasma can contain serum albumin, blood-clotting factors, immunoglobulins, lipoproteins, other proteins, and electrolytes (e.g., sodium and chloride). A crude sample comprising blood plasma can also contain blood cells. An enhancer composition described herein can be used in PCR to to aid amplification of a nucleic acid target in the presence of blood plasma.

Blood serum is generally understood as plasma from which clotting proteins have been removed, leaving mostly albumin and immunoglobulins. An enhancer composition described herein can be used in PCR to to aid amplification of a nucleic acid target in the presence of blood serum.

Full-length Taq enzyme is usually completely inhibited in a blood concentration range of about 0.004% to about 0.2% whole blood in the reaction mixture (vol/vol). Blood-resistance can be readily determined by assays described herein and known in the art (see e.g., US Pat App Pub No 2006/0084074). An enhancer composition can be used in conjunction with mutant polymerase enzymes and processes described in US Patent No. 7,462,475; US App Pub No. 2009/0170060; and WO/2008/034110.

Also provided is a method for improving performance in amplification reactions (e.g., PCR, qPCR, RT-PCR, real-time RT-PCR) containing inhibitors found in soil and soil extracts through addition of an enhancer composition described herein. Many polymerases are inhibited by substances found in soil, such as humic acid, fulvic acid, polysaccarides, and metal ions. Conventional DNA polymerase enzymes are inhibited at about 1 ng of humic acid per 50 uL reaction volume. Assays to determine the level of inhibitory substances in a sample and soil-resistance of a polymerase are known in the art. An enhancer composition can be used in conjunction with mutant polymerase enzymes and processes described in WO/2008/034110.

An enhancer composition can be used in conjunction with processes described in US Patent No. 6,403,341. An enhancer composition can be used in conjunction with processes described in US Patent No. 7,393,635. An enhancer composition can be used in conjunction with processes described in US App Pub No. 2008/0262212.

In some embodiments, an enhancer composition is used to improve performance in amplification reactions containing at least two of a dye, blood, or soil inhibitors.

### POLYMERASES

An enhancer composition described herein can be used in conjunction with a variety of polymerase enzymes. Suitable polymerases include, but are not limited to, those from polymerase families A, B, C, D, X, Y, and RT. Examples of polymerase enzymes for use with the enhancer composition of the present invention include, but are not limited to, T7 DNA polymerase; DNA Polymerase γ; *E. coli* DNA pol I; *Thermus aquaticus* pol I; *Bacillus stearothermophilus* pol I; DNA polymerase α; DNA polymerase β; DNA polymerase γ; DNA polymerase δ; DNA polymerase ε; DNA polymerase ζ; T4 polymerase; Phi29 polymerase; RB69 polymerase; DNA Polymerase III; polymerase pol β; polymerase pol σ; polymerase pol λ; polymerase pol µ; terminal deoxynucleotidyl transferase (TdT); polymerase Pol4; translesion synthesis (TLS) polymerases; Pol I; Pol II; Pol III; Pol IV; Pol V; reverse transcriptase polymerase (e.g., AMV Reverse Transcriptase, M-MLV Reverse Transcriptase, M-MLV Reverse Transcriptase, and RNase H Minus); mutations or truncations thereof, and combinations thereof.

Preferably, an enhancer composition described herein is used in conjunction with one or more thermostable polymerase including, but not limited to, Taq, Tfl, Tth, Tli, Pfu, mutations or truncations thereof, and combinations thereof.

More preferably, an enhancer composition described herein is used in conjunction with one or more polymerases selected from OmniTaq (DNA Polymerase, Inc., St. Louis, MO), Omni Klentaq (DNA Polymerase, Inc., St. Louis, MO; see KT-10 in US 7,462,475); Omni Klentaq-LA (a 50:1 mixture of Omni Klentaq with Deepven); a truncated version of wild type Taq DNA polymerase with point mutations lacking 5'→ 3' exonuclease activity (see US Patent No. 7,462,475); wild type Taq; FastStart Taq; JumpStart Taq; HotStart Plus Taq; AmpliTaq Gold; and combinations thereof.

An enhancer composition described herein can be used in conjunction with a mutant polymerase enzymes and processes described in US Patent No. 7,462,475 and US App Pub No. 2009/0170060. An enhancer composition described herein can be used in conjunction with mutant polymerase enzymes and processes described in WO/2008/034110.

### ADDITIONAL ENHANCERS

An enhancer composition described herein can be employed along with other convention amplification reaction enhancers to further improve amplification (e.g., MasterAmp™ 10X PCR Enhancer, Epicentre Biotechnologies; TaqMaster PCR Enhancer, MasterTaq Kit, PCR Extender System, 5 PRIME GmbH; Hi-Spec Additive, Bioline; PCRboost™, Biomatrica®; PCRX Enhancer System, Invitrogen; Taq Extender™ PCR Additive, Perfect Match® PCR Enhancer, Stratagene; Polymer-Aide PCR Enhancer, Sigma-Aldrich). For example, an enhancer composition can be combined with a PCR reaction mixture along with betaine (e.g., MasterAmp™ 10X PCR, Epicentre Biotechnologies). Generally, betaine alone is insufficient to overcome the inhibition of, for example, dye, blood, and/or soil when used with conventional DNA polymerases. As another example, usage of another conventional amplification reaction enhancer can be according to manufacturer instructions.

### KIT

Another aspect of the invention is directed toward kits for performance enhancement of amplification reactions. Such kits can include the enhancer compositions of the present invention and, in certain embodiments, instructions for administration. Such kits can also contain components for an amplification reaction, as described above. Preferably, a kit comprises an enhancer composition described herein, one or more polymerases (and optionally a reverse transcriptase), a buffer, and nucleotides.

Various embodiments of the kit can facilitate performance of the methods described herein, for example, PCR reactions. When supplied as a kit, the different components of the composition can be packaged in separate containers and admixed immediately before use. Components include, but are not limited to enhancer compositions described herein, primers, polymerases, deoxynucleoside triphosphates, buffer solution, divalent cations, monovalent cations, or combinations thereof. Such packaging of the components separately can, if desired, be presented in a pack or dispenser device which may contain one or more unit dosage forms containing the composition(s). The pack may, for example, comprise metal or plastic foil such as a blister pack. Such packaging of the components separately can also, in certain instances, permit long-term storage without losing activity of the components.

Kits may also include reagents in separate containers such as, for example, sterile water or saline to be added to a lyophilized active component packaged separately. For example, sealed glass ampules may contain lyophilized agent(s) and in a separate ampule, sterile water or sterile saline, each of which has been packaged under a neutral non-reacting gas, such as nitrogen. Ampules may consist of any suitable material, such as glass, organic polymers, such as polycarbonate, polystyrene, ceramic, metal or any other material typically employed to hold reagents. Other examples of suitable containers include bottles that may be fabricated from similar substances as ampules, and envelopes that may consist of foil-lined interiors, such as aluminum or an alloy. Other containers include test tubes, vials, flasks, bottles, syringes, and the like. Containers may have a sterile access port, such as a bottle having a stopper that can be pierced by a hypodermic injection needle. Other containers may have two compartments that are separated by a readily removable membrane that upon removal permits the components to mix. Removable membranes may be glass, plastic, rubber, and the like.

In certain embodiments, kits can be supplied with instructional materials. Instructions may be printed on paper or other substrate, or may be supplied as an electronic-readable medium, such as a floppy disc, mini-CD-ROM, CD-ROM, DVD-ROM, Zip disc, videotape, audio tape, and the like. Detailed instructions may not be physically associated with the kit; instead, a user may be directed to an Internet web site specified by the manufacturer or distributor of the kit.

In some embodiments, the numbers expressing quantities of ingredients, properties such as molecular weight, reaction conditions, and so forth, used to describe and claim certain embodiments of the invention are to be understood as being modified in some instances by the term "about." Accordingly, in some embodiments, the numerical parameters set forth in the written description and attached claims are approximations that can vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the invention are approximations, the numerical values set forth in the specific examples are reported as precisely as practicable. The numerical values presented in some embodiments of the invention may contain certain errors necessarily resulting from the standard deviation found in their respective testing measurements.

In some embodiments, the terms "a" and "an" and "the" and similar references used in the context of describing a particular embodiment of the invention (especially in the context of certain of the following claims) can be construed to cover both the singular and the plural. The recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g. "such as") provided with respect to certain embodiments herein is intended merely to better illuminate the invention and does not pose a limitation on the scope of the invention otherwise claimed. No language in the specification should be construed as indicating any non-claimed element essential to the practice of the invention.

Groupings of alternative elements or embodiments of the invention disclosed herein are not to be construed as limitations. Each group member can be referred to and claimed individually or in any combination with other members of the group or other elements found herein. One or more members of a group can be included in, or deleted from, a group for reasons of convenience or patentability. When any such inclusion or deletion occurs, the specification is herein deemed to contain the group as modified thus fulfilling the written description of all Markush groups used in the appended claims.

Having described the invention in detail, it will be apparent that modifications, variations, and equivalent embodiments are possible without departing the scope of the invention defined in the appended claims. Furthermore, it should be appreciated that all examples in the present disclosure are provided as non-limiting examples.

### EXAMPLES

The following non-limiting examples are provided to further illustrate the present invention. It should be appreciated by those of skill in the art that the techniques disclosed in the examples that follow represent approaches the inventors have found function well in the practice of the invention, and thus can be considered to constitute examples of modes for its practice.

### EXAMPLE 1

The following primer oligonucleotideswere used in the PCR reaction to amplify a 630 bp CCR5 target (see e.g., FIG. 1A) and a 488 bp HRES-1-c fragment from 10% whole blood (see e.g., FIG. 1 B) or from 0%-25% diluted whole blood (see e.g., FIG. 1C): forward 5'-GCAGCGGCAGGACCAGCCCCAAGATGACTATCT-3', (SEQ ID NO:1) and reverse 5'-TGGAACAAGATGGATTATCAAGTGTCAAGTCCA-3' (CCR5; SEQ ID NO:2); forward 5'-TCCGGCCGCGCCACCGCCACCCTCA-3' (SEQ ID NO:3), and reverse 5'-ACCGCCAGAGCGCCCAGCCCGCGCA-3' (HRES-1-c; SEQ ID NO:4). The final concentration of each primer was 0.2 µM used in a common master mix. The amount of enzyme used was 2 units per 50 µl reaction. OT was challenged with 10% blood in the presence of different formulations of PEC (see e.g., FIG. 1A, 1B). OT and OKT were challenged with 0% (4 ng DNA) to 25% whole blood in the presence of trehalose, L-carnitine, NP-40 and PEC, respectively, but the control contained no enhancer (see e.g., FIG. 1 C). After 40 cycles the products were analyzed in 1.5% agarose gel electrophoresis (see e.g., FIG. 1A, 1B and 1C).

Results showed that when the concentration of NP-40 and L-carnitine were constant, the optimized working concentration of trehalose was 0.5-0.8 M; when the concentrations of NP-40 and trehalose were constant, the optimized working concentration of L-carnitine was 0.12-0.72 M for this non GC-rich target (see e.g., FIG. 1 A). When the concentration of NP-40 and L-carnitine were constant, the optimized working concentration of trehalose was 0.4-0.8 M; when the concentrations of NP-40 and trehalose were constant, the optimized working concentration of L-carnitine was 0.48-1.2 M for this GC-rich target (see e.g., FIG. 1 B). OT and OKT were able to amplify this GC-rich target from purified DNA and different concentrations of blood only with PEC. Without PEC, OT and OKT obtained either no products or non-specific products (see e.g., FIG. 1 C).

Based on these results, a PCR enhancer cocktail was formulated to that contain trehalose 0.6 M, L-carnitine 0.48 M and NP-40 0.8%, which can work on both non-GC-rich and GC-rich targets.

### EXAMPLE 2

The impact of PEC on PCR specificity was assessed by direct amplification of a 488 bp fragment of HRES1-1-c target from blood samples in absence or presence of PEC. 1.3 M of Betaine (Sigma Aldrich, Cat No B0300), a known PCR enhancer, was used as a comparison. Furthermore, the impact of PEC on PCR sensitivity was evaluated by amplifying the same target from both purified DNA and blood. The following primer oligonucleotides were used in the PCR reaction to amplify this target: forward 5' TCCGGCCGCGCCACCGCCACCCTCA-3' (SEQ ID NO:3), and reverse 5'-ACCGCCAGAGCGCCCAGCCCGCGCA-3' (SEQ ID NO:4). The blood sample was a five-fold series diluted from 4% to 0.00001% (see e.g., FIG. 2, lanes 1-9) and the negative control contained no DNA or blood (N). This target (GC content is 81%) was amplified from different concentrations of the blood sample (see e.g, FIG. 2A). Furthermore, this target was amplified from different concentrations of the blood sample or the same amount of purified DNA in the presence of PEC (see e.g., FIG. 2B). The final concentration of each primer was 0.2 µM used in a common master mix. The amount of each enzyme used was 2 units per 50 µl reaction. PCR products were resolved in 1.5% agarose gel electrophoresis (see e.g., FIG. 2).

Results showed that OT was able to amplify this target from different concentrations of blood samples with a specific products and higher yield in the presence of PEC. With Betaine, OT obtained either lower yields or non-specific products. The PCR sensitivity and specificity from blood samples are very similar to that from purified DNA when using Betaine.

### EXAMPLE 3

The following primer oligonucleotides were used in the PCR reaction to amplify a 630 bp of CCR5 target and a 488 bp of HRES-1-c from purified DNA (4 ng) and 1.25-25% of whole blood with or without PEC: forward 5'-GCAGCGGCAGGACCAGCCCCAAGATGACTATCT-3' (SEQ ID NO;1), and reverse 5'-TGGAACAAGATGGATTATCAAGTGTCAAGTCCA-3' (CCR5; SEQ ID NO:2)); forward 5'-TCCGGCCGCGCCACCGCCACCCTCA-3' (SEQ ID NO:3), and reverse 5'-ACCGCCAGAGCGCCCAGCCCGCGCA-3' (HRES-1-c; SEQ ID NO;4). The final concentration of each primer was 0.2 µM used in a common master mix. The amount of enzyme used was 2 units per 50 µl reaction. After 40 cycles the products were analyzed in 1.5% agarose gel electrophoresis (see e.g., FIG. 3).

Results showed that OT and OKT were only able to amplify the CCR5 target from DNA, but not in blood with the absence of PEC. However, OT and OKT were able to amplify the CCR5 target from both purified DNA and different concentrations of blood in the presence of PEC. OT and OKT were not able to amplify the HRES-1-c target from both DNA and blood in absence of PEC; however, specific products could be obtained from both DNA and blood in presence of PEC.

### EXAMPLE 4

The following primer oligonucleotides were used in the PCR reaction to amplify a 300 bp target of β-actin and a HRES-1-c fragment from purified DNA (4 ng) and 1.25-25% of whole blood treated with different anticoagulant in presence or absence of PEC: forward 5'-TCACCCACACTGTGCCCATCTACGA -3' (SEQ ID NO:5), and reverse 5'-CAGCGGAACCGCTCATTGCCAATGG -3' (β-actin; SEQ ID NO:6); forward 5'-TCCGGCCGCGCCACCGCCACCCTCA-3' (SEQ ID NO:3), and reverse 5'-ACCGCCAGAGCGCCCAGCCCGCGCA-3' (HRES-1-c; SEQ ID NO:4). The final concentration of each primer was 0.2 µM used in a common master mix. The amount of enzyme used was 2 units per 50 µl reaction. After 40 cycles the products were analyzed in 1.5% agarose gel electrophoresis (see e.g., FIGs. 4A, 4B).

Results showed that OT and OKT were able to amplify the 300 bp of β-actin target and the 488 bp target of HRES-1-c from a series dilution of blood samples treated with different anticoagulants in the presence of PEC. Without PEC, OKT was only able to amplify β-actin from lower concentrations of blood. PEC enhanced the reaction and enabled OT and OKT to tolerate at least 25% of blood (see e.g., FIG. 4A). OT-LA and OKT-LA could not amplify HRES-1-c without PEC; however, they were able to amplify this target and to tolerate at least 25% of blood in presence of PEC (see e.g. FIG. 4B).

### EXAMPLE 5

A 210 bp target of Anthrax Bacillus gene (GC content is 33%), 690 bp of duck hepatitis B P gene (DHBV-P, GC content is 44%), 630 bp of CCR5 (GC content is 47%), 980 bp of SLC19A (GC content is 74%) and 488 bp of HRES-1-c (GC content is 81 %) were amplified directly from 10% blood sample using OT-LA or OKT in the absence or presence of PEC. 1.3 M Betaine (Sigma Aldrich, Cat No B0300) was included as a comparison. The following primer oligonucleotides were used in the PCR reaction to amplify these endogenous and exogenous targets: forward 5'-ATCACCAGAGGCAAGACACCC-3' (SEQ ID NO:7), and reverse 5'-ACCAATATCAAAGAACGACGC-3' (Anthrax; SEQ ID NO:8); forward 5'-ATGCCCCAACCATTGAAGCAATC-3' (SEQ ID NO:9), and reverse 5'-ACGTCCATTGATTTTGCTGGATG-3' (DHBVP; SEQ ID NO:10); forward 5'-GCAGCGGCAGGACCAGCCCCAAGATGACTATCT-3' (SEQ ID NO:1), and reverse 5'-TGGAACAAGATGGATTATCAAGTGTCAAGTCCA-3' (CCR5; SEQ ID NO:2); forward 5'-CCTTCTGTTCTGTGCAGTGG-3' (SEQ ID NO:11), and reverse 5'-CGGACTCCGGGACTACAG-3' (SLC19A; SEQ ID NO:12); forward 5'-TCCGGCCGCGCCACCGCCACCCTCA-3' (SEQ ID NO:3), and reverse 5'-ACCGCCAGAGCGCCCAGCCCGCGCA-3' (HRES-1-c; SEQ ID NO:4). The products were resolved in 1.5% agarose gel (see e.g., FIG. 5). Lane1 indicated 1.3 M of Betaine; lane 2, No enhancer; lane 3, PEC; lane 4, negative control (no DNA or blood).

Results showed that PEC worked well on direct amplification of both of the endogenous and exogenous targets, as well as the non-GC-rich and GC-rich targets (see e.g., FIG. 5).

### EXAMPLE 6

The following primer oligonucleotides were used in the PCR reaction to amplify a 1202 bp target of SIM2 (lane 1), 1185 bp target of DIP2A (lane 2) and 980 bp target of SLC19A1 (lane 3) from purified human genome DNA or 5% whole blood using FastStart (Roche) with GC solution, HotStart Plus (Qiagnen) with Q solution, Taq (NEB) with Hi Spec Additive (Bioline), and OT-and OKT with PEC (see e.g., FIG. 6). Negative control did not contain DNA or blood (N). Forward 5'-CCGTTTTCACGTGTGTGTGT -3' (SEQ ID NO:13), and reverse 5'-TTCCTTCTCCCTCCTGGTCT -3' (SIM2; SEQ ID NO:14); forward 5'-AGGGGAAGGAAGCAGGACT-3' (SEQ ID NO:15), and reverse 5'-CAGCTCAGCCAGGCTCTC-3' (DIP2A; SEQ ID NO:16); forward 5'-CCTTCTGTTCTGTGCAGTGG-3' (SEQ ID NO:11), and reverse 5'-CGGACTCCGGGACTACAG-3' (SLC19A1; SEQ ID NO:12). The final concentration of each primer was 0.2 µM used in a common master mix. The amount of enzyme used was 2 units per 50 µl reaction. After 40 cycles the products were analyzed in 1.5% agarose gel electrophoresis (see e.g., FIG. 6A, 6B).

Results showed that not all of the enzymes could obtain specific products for these GC-rich targets without a PCR enhancer (see e.g., FIG. 6A). OT and OKT obtained very specific amplicons from both purified DNA and blood sample in the presence of PEC. FastStart received expected products accompanied with non-specific bands for purified DNA but failed in the blood sample. HotStart Plus yielded only a week band for DIP2A from the blood sample and Taq (NEB) totally failed (see e.g., FIG. 6B).

### EXAMPLE 7

The following primer oligonucleotides were used in the PCR reaction to amplify a 488 bp fragment of HRES-1-c from purified human genome DNA and 5% whole blood using different DNA polymerase with PEC or with other commercial enhancers: forward 5'-TCCGGCCGCGCCACCGCCACCCTCA-3' (SEQ ID NO:3), and reverse 5'-ACCGCCAGAGCGCCCAGCCCGCGCA-3' (SEQ ID NO:4). The final concentration of each primer was 0.2 µM used in a common master mix. The amount of each enzyme used was 2 units per 50 µl reaction. After 40 cycles the products were analyzed in 1.5% agarose gel electrophoresis (see e.g., FIG. 7).

Results showed that FastStart, AmpliTaq Gold and JumpStart obtained a product only in the DNA sample, and Taq (NEB) failed in both the DNA and the blood sample in the presence of the corresponding enhancer. However, in the presence of PEC, these enzymes received specific amplicons from both DNA and blood except for AmpliTaq Gold.

### EXAMPLE 8

The following primer oligonucleotides were used in the PCR reaction to amplify a 210 bp fragment of Anthrax Bacillus from series diluted soil extracts using different DNA polymerase and enhancers: forward 5'-ATCACCAGAGGCAAGACACCC-3' (SEQ ID NO:7), and reverse 5'-ACCAATATCAAAGAACGACGC-3' (SEQ ID NO:8). Soil extract was five-fold diluted from 0 to 1:40 (see e.g., lane 1 to 5, FIG. 8) and spiked with Anthrax Bacillus. 210 bp pag gene of Anthrax Bacillus was amplified using different DNA polymerase in the absence (see e.g., FIG. 8A) or presence of PCR enhancer (see e.g., FIG. 8B). Positive control contained Anthrax Bacillus but without soil extract (lane 6). FastStart (Roche) was applied with GC solution. HotStart Plus (Qiagnen) with Q solution, Taq (NEB) with Hi Spec Additive (Bioline), and OT and OKT with PEC. The final concentration of each primer was 0.2 µM used in a common master mix. The amount of each enzyme used was 2 units per 50 µl reaction. After 40 cycles the products were analyzed in 1.5% agarose gel electrophoresis (see e.g., FIG. 8A, 8B).

Results showed that in the absence of the enhancer, OKT was able to obtain product from 1:10 dilution, FastStart yielded a product from 1:20 dilution and HotStart Plus obtained product from 1:40 dilution. JumpStart, Taq (NEB) and OT could not obtain amplicons (see e.g., FIG. 8A); in the presence of the enhancer, OT and OKT were able to amplify the target from 1:5 dilution with high yield. FastStart was able to amplify the target from a 1:10 dilution and HotStart received the product from a 1:20 dilution with a very faint band. JumpStart and Taq (NEB) failed (see e.g., FIG. 8B).

### EXAMPLE 9

The following primer oligonucleotides were used in the real time PCR reaction to amplify a 281 bp mousepox virus gene from 5% whole blood in the presence and absence of PEC using OT (see e.g., FIGs. 9A, 9B) or OKT (see e.g., FIGs. 9C, 9D): forward 5'-AGAAGATATCAGACGATCCACAATC-3' (SEQ ID NO:17), and reverse 5'-GTAGAACGACGCCAGAATAAGAATA-3' (SEQ ID NO:18). The supernatant of mousepox virus cell culture was 5 times fold diluted and mixed with 5% EDTA treated whole blood and the 281 bp mousepox virus gene was amplified from these samples using OT (see e.g., FIGs. 9A, 9B) or OKT (see e.g., FIGs. 9C, 9D) in the presence or absence of PEC. The samples were run for 40 cycles in Option 2 real-time cycler and 10X SYBR green dye was applied in each reaction. Negative control was included. (see e.g. FIGs. (a, 9B, 9C, 9D).

Results showed that OT and OKT was able to amplify 281 bp of mousepox virus gene from 5% whole blood containing different concentrations of templates in presence of PEC with a nice curve (see e.g., FIGs. 9A, 9C). Without PEC OT was not able to amplify this target (see e.g., FIG. 9B). OKT was able to amplify this target in absence of PEC but with delay Ct value and lower signal comparing in presence of PEC (see e.g., FIG. 9D).

### EXAMPLE 10

The following primer oligonucleotides were used in real time PCR reactions to amplify a 281 bp mousepox virus gene from 10% heparin treated plasma in the presence of PEC using OT/OKT mixture: forward 5'-AGAAGATATCAGACGATCCACAATC-3' (SEQ ID NO:17), and reverse 5'-GTAGAACGACGCCAGAATAAGAATA -3' (SEQ ID NO:18). The supernatant of mousepox virus cell culture was 10 fold diluted and mixed with 10% heparin treated plasma and the 281 bp mousepox virus gene was amplified from these samples using OT/OKT 1:1 mixture in the presence of PEC. The samples were run for 45 cycles in an Option 2 real-time cycler and a 200 nM TET labeled TaqMan probe was applied in each reaction. Negative control was included (see.e.g., FIG. 10).

Results showed that OT/OKT was able to amplify a 281 target bp of mousepox virus gene from 10% heparin treated plasma containing different concentrations of templates in the presence of PEC with a proportion curve.

### EXAMPLE 11

The following primer oligonucleotides were used in real time PCR reactions to amplify a 281 bp mousepox virus gene from 10% plasma or 10% serum in the presence of PEC or in the presence of PECplus using OT/OKT mixture: forward 5'-AGAAGATATCAGACGATCCACAATC-3' (SEQ ID NO:17), and reverse 5'-GTAGAACGACGCCAGAATAAGAATA-3' (SEQ ID NO:18). The supernatant of the mousepox virus cell culture was 10 times fold diluted and mixed with 10% plasma treated with heparin, citrate and EDTA or 10% human serum. A 281 bp mousepox virus gene was amplified from these samples using OT/OKT 1:1 mixture in presence of PEC (see e.g., FIG. 11A) or in presence of PECplus (see e.g., FIG. 11 B). The samples were run for 45 cycles in an Option 2 real-time cycler and a 200 nM TET labeled TaqMan probe was applied in each reaction (see.e.g., FIGs. 11A, 11B).

Results showed that the OT/OKT mixture was only able to amplify 281 bp of mousepox virus gene from 10% heparin treated plasma but could not amplify this target from citrate and EDTA treated plasma and serum in presence of PEC (see e.g., FIG. 11 A). However, in the presence of PEC plus, it was able to amplify this target from all the differently treated plasma and serum (see e.g., FIG. 11 B).

### EXAMPLE 12

The following primer oligonucleotides were used in PCR reaction to amplify a 244 bp influenza virus gene from purified RNA (see e.g., FIG. 12, lane 1), 10% citrate-treated plasma (see e.g., FIG. 12, lane 2), 10% serum (see e.g., FIG. 12, lane 3) and 10% whole blood (see e.g., FIG. 12, lane 4) in the absence or in the presence of PECplus.: forward 5'-CTTCTAACCGAGGTCGAAACG-3' (SEQ ID NO:19), and reverse 5'-AGGGCATTTTGGACAAARCGTCTA-3' (SEQ ID NO:20). The purified virus RNA was mixed with 10% plasma, serum and whole blood and 244 bp influenza virus target was amplified from these samples by one-step RT-PCR using MMLV/OT/OKT mixture. The samples were run for 45 cycles and the products were analyzed in 1.5% agarose gel electrophoresis (see e.g., FIG. 12).

Results showed that MMLV/OT/OKT mixture was only able to amplify this target from RNA in absence of PECplus. Hower, in presence of PECplus, it was able to amplify this target from both purified RNA sample and all other crude samples.

### EXAMPLE 13

The following example demonstrates RT-PCR detection of HCV1 b from virus-like particles (armored RNA) in crude samples using an enhancer composition of the invention. Provided above are data from one-step RT-PCR detection of an influenza virus gene in crude samples. For the present example, a combination ofw.t. MMLV reverse transcriptase (MMLV-H+) and OmniTaq/Omni Klentaq enzyme mix was used to amplify the target from naked virus RNA mixed with blood or blood fractions in the reactions.

The enzyme mix was composed of 200 units MMLV reverse transcriptase RNase H minus (MMLV-H⁻), 2 units of OmniTaq polymerase (DNA Polymerase, Inc., St. Louis, MO) and 5 units Omni Klentaq polymerase (DNA Polymerase, Inc., St. Louis, MO). The armored HCV1b RNA (AsuraGen, www.asuragen.com) was spiked in human serum, plasma, and whole blood that were treated with EDTA, citrate, or heparin. The armored RNA is a complex of MS2 bacteriophage coat protein and viral RNA produced in Escherichia coli by the induction of an expression plasmid that encodes the coat protein and the RNA sequence. These virus-like particles containing coated protein and target sequence are RNase-resistant, noninfectious, and are stable in human plasma at 40C >300 days. They are good candidates as RNA controls and standards in RNA virus detection and are better than naked RNA model to mimic clinical pathogen detection in crude samples.

RT-PCR reactions contained 50 copies of intact armored RNA and 10% serum, plasma, or whole blood. RT-PCR reactions also contained an enhancer composition containing 0.64 M trehalose, 0.12 M L-carnitine, 0.4% NP-40 and 10 u heparin per ml serum, plasma, or whole blood (PEC-Plus). Naked RNA (obtained after the HCV armored RNA was heated at 75° for 5 min to release RNA) and intact armored RNA particles without addition of blood or blood components were included as positive controls. Reactions that contained no RNA or no reverse transcriptase were included as negative controls as well. The one-step RT-PCR reactions were performed for 40 cycles in a PTC-200 thermal cycler and a 244 bp target of the 5'UTR region of HCV1b was amplified from naked RNA, armored RNA, and crude samples in the presence of the enhancer cocktail. Fifteen µl of PCR product of each reaction was loaded in 2% agarose gel for electrophoresis.

Results showed successful amplification of a 244 bp target of the 5'UTR region of HCV1b from crude samples, without extracting RNA, using armored RNA and MMLV-H- blended with OmniTaq and Omni Klentaq in the RT-PCR reactions (see e.g., FIG. 13).

MMLV-H- can perform in a higher temperature (50-55°C) than MMLV-H+ (37-40°C), which may provide a benefit for GC-rich targets, as well as for disrupting the encapsidated protein coat and envelope of viruses or cell membrane.

Results demonstrated that the PCR inhibition resistant enzymes OmniTaq and Omni Klentaq, blended with MMLV- H- in the presence of the PCR enhancer cocktail, were able to specifically amplify the HCV target both from naked RNA and intact armored RNA virus-like particles in crude samples containing blood, serum or plasma (see e.g., FIG. 13). The negative controls showed only primer dimers, indicating that the specific products were amplified from RNA rather than from DNA contaminations.

Results also showed that the enhancer cocktail works on RNA targets with other commercial Taqs and reverse transcriptases, which demonstrated this enhancer cocktail provides a general stimulating effect on RT-PCR (data not shown). These data also suggest that some components in the enhancer may inhibit ribonucleases present in crude samples; relieve the inhibition by some PCR inhibitors such as Ig G, lacoferrin, heme, as well as some reverse transcriptase inhibitors; and aid in disrupting the virus protein envelope.

Furthermore, in the presence of enhancer cocktail, both Omnitaq and Omni Klentaq enzymes were able to tolerate at least 20% plasma and serum, and 15% whole blood. In addition, they can tolerate different anticoagulants that are commonly used in clinical diagnostics. Thus, the enhancer cocktail can also be applied in analysis of mRNA expression in crude sample without RNA isolation prior to RT-PCR.

### EXAMPLE 14

The following example demonstrates performance of the PCR enhancer cocktail with OmniTaq and Omni Klentaq in real-time RT-PCR TaqMan assay in crude sample.

An enzyme mix of 100 units MMLV RT, 1 unit OmniTaq (DNA Polymerase, Inc., St. Louis, MO) and 2.5 units Omni Klentaq (DNA Polymerase, Inc., St. Louis, MO) was used to amplify a 244 bp influenza virus RNA target in the presence of the PCR enhancer cocktail and 200 mM TaqMan probe. Purified influenza virus RNA was 10-fold diluted 7 times and spiked in human serum. The one-step RT-PCR reactions (25 µl) contained different concentrations of RNA and constant concentrations of human serum (5%) (see e.g., FIG. 14A). Parallel reactions with purified RNA alone were included as a comparison and positive controls (see e.g., FIG. 14B). RT-PCR reactions also contained an enhancer composition containing 0.64 M trehalose, 0.12 M L-carnitine, 0.4% NP-40 and 10 u heparin per ml serum (PEC-Plus). The reactions were performed in an Opticon-2 real-time PCR cycler. The fluorescence signal was detected after the annealing step.

Results showed successful amplification of 244 bp of influenza virus gene from mimic (model) clinical samples containing 5% human serum using combination of OmniTaq/Omni Klentaq and MMLV-RT in the presence of the enhancer cocktail. The fluorescent signal in crude samples was relatively lower than these in purified RNA, however, the sensitivity was not compromised, as compared to the results with purified RNA.

Results also showed successful amplification of RNA targets from plasma and blood in the presence of the enhancer cocktail (data not shown). The optimal concentrations of TaqMan probe were 200-400 mM for 5-20% plasma and serum, and 400-800 mM for 5-10% whole blood.

Thus, the enhancer cocktail can be used to facilitate PCR amplification of DNA targets and RT-PCR amplification of RNA targets with a variety of commercial polymerases (and reverse transcriptases) in crude samples, such as crude clinical samples.

### EXAMPLE 15

The following example demonstrates performance of a heparin-containing PCR enhancer cocktail.

Citrate, EDTA, and heparin are common anticoagulants used for blood treatment in diagnostics. An inhibitory effect of heparin on Taq in PCR with purified DNA or RNA substrate has been documented (see e.g., Chen et al. 1990 Nucleic Acids Res 18, 3255-3260; Satsangi et al. 1994 Lancet 343, 1509-1510; Poli et al. 1993 PCR Methods Appl. 2, 356-8; Wang et al. 1992 J Clin Microbiol. 303, 750-753). Data presented herein demonstrate that heparin alone or combined with other chemicals helps PCR in crude blood samples (e.g., plasma or serum) with all anticoagulants.

A 0.5 kb human genomic target (endogenous target) was directly amplified from heparin treated plasma, citrate treated plasma, EDTA treated plasma, and serum using Omni Klentaq. The reactions were performed in the presence of heparin alone, heparin combined with D-(+)-trehalose, heparin combined with L-carnitine, heparin combined with NP-40, and heparin combined with PEC (i.e., PEC-Plus). The reactions containing no PCR enhancer were included as a control. The PCR products were resolved in 1.5% agarose gel and stained with ethidium bromide.

Results showed that higher yield was obtained in the presence of heparin plus trehalose or heparin plus PEC (see e.g., FIG. 15). The more specific products were observed in the presence of heparin plus L-carnitine (see e.g., FIG. 15). In the absence of heparin, PCR failed to detect the target from the crude samples, except with heparin treated plasma (i.e., already containing heparin) (see e.g., FIG. 15).

A 189 bp target of mousepox virus gene was amplified by Omni Taq/Omni Klentaq mix (1:1) from 0.1 ng viral total DNA and crude sample spiked with virus including 10% heparin, citrate treated plasma, EDTA treated plasma, serum, and EDTA treated whole blood. The reactions were performed in the absence of enhancer, and in the presence of heparin alone, combination of heparin and trehalose, PEC, and PEC-Plus (i.e., PEC plus heparin). Negative controls contained no virus DNA (N).

Results showed that heparin alone was able to reverse the inhibitory effect of plasma treated with citrate and EDTA (see e.g., FIG. 16, Heparin, lanes 3 and 4, respectively) but did not reverse inhibition by whole blood (see e.g., FIG. 16, Heparin, lane 6). Furthermore, the combination of heparin and D-(+)-trehalose was effective in relieving the inhibitory effect of whole blood, plasma, and serum (see e.g., FIG. 16, Heparin + trehalose, compare lanes 6, 2-4, and 5, respectively); however, it inhibited the amplification of purified DNA (see e.g., FIG. 16, Heparin + trehalose, lane 1). In the presence of PEC alone, the target was amplified from purified DNA, heparin treated plasma, and whole blood (see e.g., FIG. 16, PEC, lanes 1, 2, and 6, respectively), but not from citrate treated plasma, EDTA treated plasma, or serum (see e.g., FIG. 16, PEC, lanes 3, 4, and 5).

Finally, only with the combination of PEC plus heparin (at concentration 5-20 U/ml plasma, serum and whole blood) was the target amplified from DNA and all crude samples tested (see e.g., FIG. 16, PECplus, lanes 1-6).

The above results demonstrate that heparin alone is inhibitory to purified DNA as compared to heparin combined with the other PEC components. Without being bound to one interpretation and in no way limiting the present invention, it is presently thought that trehalose or carnitine relieve or compensate for the inhibitory effect of heparin on amplification of purified DNA.

Plasma and serum contain less heme/hemoglobin than whole blood and should have a less inhibitory effect on PCR performance than whole blood. Surprisingly, it was observed that OmniTaq (FL-22) and Omni Klentaq (Klentaq-10) could amplify targets from whole blood treated with three anticoagulants, while, with some targets, they were only active in heparin treated plasma, not in the citrate and EDTA treated plasma. Thus heparin may play a role in overcoming PCR inhibition. Indeed, heparin alone was able to relieve the inhibitory action at least in citrate and EDTA treated plasma samples as demonstrated herein, and adding heparin to PEC (PEC-Plus) allowed efficient amplification from all crude specimens by OmniTaq and Omni Klentaq.

In additional pull-down experiments with heparin-conjugated beads, it was demonstrated that heparin indeed binds some potent PCR inhibitors, such as IgG (immunoglobulins) and lactoferrin (results not shown).

### EXAMPLE 16

The following example demonstrates performance of a heparin-containing PCR enhancer cocktail in real-time PCR TaqMan assays with crude samples.

A 189 bp target of mousepox virus gene was amplified from different anticoagulant-treated plasma or serum by an OmniTaq and Omni Klentaq mix using the TaqMan assay in the absence or the presence of 10 units heparin / ml plasma or serum (see e.g., FIG. 17).

Results showed that the target could only be amplified from heparin-treated plasma but not from other coagulant-treated plasma or serum in the absence of an addition of heparin (see e.g., FIG. 17A). But this target could be detected from all coagulant-treated plasma and serum in the presence of heparin (see e.g., FIG. 17B).

### EXAMPLE 17

The following example demonstrates performance of a heparin-containing PCR enhancer cocktail in RT-PCR assays with crude samples.

A 144 nt RNA target of influenza virus was amplified by 2 units of OmniTaq/KlenTaq mix (1:1) and MMLV RT from 8% (final concentration in one-step RT-PCR) heparin-treated plasma, citrate-treated plasma, serum, and heparin-treated whole blood (see e.g., FIG. 18, lanes 1-4, respectively). The reactions were performed in the presence of PEC or PEC-Plus (PEC plus heparin). The PCR products were resolved in 2% agarose gel and stained with ethidium bromide.

Results showed that this one-step RT-PCR system was able to amplify the virus target from all crude samples in the presence of PEC-Plus (see e.g., FIG. 18, PEC-Plus, lanes 1-4). But in the presence of PEC (i.e., no heparin), the one-step RT-PCR system only amplified the target from heparin treated plasma and blood (see e.g., FIG. 18, PEC, lanes 1 and 4).

### EXAMPLE 18

The following example demonstrates performance of a PCR enhancer cocktail containing NP-40 or Brij-58 in conventional PCR.

PCR was performed in a 50 µl volume in 0.2 ml tubes. Each reaction contained enhancer treatment or control along with reaction buffer, polymerase enzyyme, primers, dNTPs, and target sample.

The enhancer formulation included 0.6 M D-(+)-Trehalose, 0.2 M L-carnitine, and 0.4% NP-40 or 0.1% Brij-58 (final concentration in the PCR). Reactions were run with Lane 1: No enhancer (see e.g., FIG 19, lane 1); PEC containing 0.4% NP-40 (see e.g., FIG 19, lane 2); PEC containing 0.1% Brij-58 (see e.g., FIG 19, lane 3).

The OmniTaq reaction buffer I contained 50 mM Tris-HCl pH 8.7, 10 mM ammonium sulfate, 0.1% (v/v) Tween 20, and 1.5 mM magnesium chloride. The OmniTaq reaction buffer II contained the same content as buffer I except using 0.1 % Brij-58 to substitute for 0.1 % Tween 20. The Omni Klentaq reaction buffer I contained 50mM Tris-HCl pH 9.2, 16 mM ammonium sulfate, 0.1% (v/v) Tween 20, and 2.5 mM magnesium chloride. The Omni Klentaq reaction buffer II contained the same components as buffer I except the use of 0.1 % Brij-58 instead of 0.1% Tween 20.

The reaction contained 2 U of OT or OKT enzyme, 0.2 µmol each primer, and 200 µmol dNTP mix for DNA samples. The amount of OT or OKT for blood samples was 10 times higher than that for DNA samples. The following primer oligonucleotides were used in the PCR reaction to amplify this target: forward 5'-ATGCCCCAACCATTGAAGCAATC-3' (SEQ ID NO:9), and reverse 5'-ACGTCCATTGATTTTGCTGGATG-3' (SEQ ID NO:10). The reactions were performed in PTC-200 Peltier Thermocycler with an initial denaturation for 6 min at 94 °C followed by the thermal cycles as follows: denaturation step at 94 °C for 30 sec, annealing step at 52 °C for 45 sec, and an elongation step at 70 °C for 2 min for 35 cycles.

The results showed that PEC significantly enhanced PCR, especially in samples containing blood (see e.g., FIG. 19, comparing blood lane 1 with blood lanes 2 or 3), and the performance of PEC containing Briji-58 and PEC containing NP-40 was very similar in both buffer conditions (see e.g., FIG. 19, comparing lane 3 with lane 2). Thus, Brij-58 can be used as a nonionic detergent component of PEC in PCR protocols.

Additonal testing was performed on a series of enhancer formulations containing 0.6 M Trehalose and 0.2 M L-carnitine, mixed with 0, 0.02%, 0.04%, 0.06%, 0.08%, 0.1%, 0.2%, 0.3%, 0.4% and 0.5% of Brij-58 (final concentration in PCR provided through PEC). Results showed that all of the above formulations of PEC (even without Brij) significantly enhance the PCR reactions, especially for crude samples. The enhancement effect, however, was stronger in the presence of detergent, and the optimal concentrations of Briji in the PEC were 0.04% - 0.2%.

### EXAMPLE 19

The following example demonstrates performance of a PCR enhancer cocktail containing NP-40 or Brij-58 in real time PCR with SYBR Green detection.

Real-time PCR was performed with a 25 µl volume in 0.2 ml tubes. Each reaction contained enhancer treatment or control along with reaction buffer, dye, polymerase enzyyme, primers, dNTPs, and target sample.

The enhancer formulation included 0.6 M D-(+)-Trehalose, 0.2 M L-carnitine, and 0.4% NP-40, 0.1% Brij-58, or 0.04% Brij-58 (final concentration in the PCR). Reactions were run with: n0 enhancer; PEC containing 0.4% NP-40; PEC containing 0.1% Brij-58; or PEC containing 0.04% Brij-58 (see e.g., FIG 20).

The Omni Klentaq reaction buffer contained 50 mM Tris-HCl pH 9.2, 16 mM ammonium sulfate, 0.1% (v/v) Tween 20, and 2.5 mM magnesium chloride.

The reaction contained 10 U of OKT enzyme, 0.2 µmol of each primer, 200 µmol of dNTP mix, 1.25 µl of heparin-treated blood (5% final concentration in PCR), 1 µl of 1:125 diluted duck serum containing 690 bp target of duck hepatitis B P virus gene (DHBVP) and 20X SYBR green dye. The following primer oligonucleotides were used in the real-time PCR reaction to amplify this target: forward 5'-ATGCCCCAACCATTGAAGCAATC-3' (SEQ ID NO:9), and reverse 5'-ACGTCCATTGATTTTGCTGGATG-3' (SEQ ID NO:10). The qPCR was performed in an Opticon-2 cycler (BioRad, CA) with an initial denaturation for 6 min at 94°C followed by a denaturation step at 94°C for 30 sec, annealing at 52°C for 45 sec, and an elongation at 70°C for 2 min for 40 cycles. Fluorescence was detected at each annealing step.

Results indicated that PEC containing Brij-58 had a similar performance on PCR and Ct values compared to PEC containing NP-40.

The results showed that PEC significantly enhanced real time PCR in samples containing blood (see e.g., FIG. 20, comparing no enhancer to PEC treatments), and the performance of PEC containing 0.1 % Briji-58 and PEC containing 0.4% NP-40 was very similar while PEC containing 0.04% Briji-58 had enhanced performance (see e.g., FIG. 19, comparing lane 3 with lane 2). Thus, Brij-58 can be used as a nonionic detergent component of PEC in real time PCR protocols.

## Claims

1. A composition for enhancing nucleic acid amplification comprising:
trehalose;
carnitine;
a non-ionic detergent; and
optionally, heparin.

2. The composition of claim 1 comprising:
0.1 to 1.0 M D-(+)-trehalose per amplification reaction mixture volume;
0.1 to 1.5 M L-carnitine per amplification reaction mixture volume;
0.01% to 8% non-ionic detergent per amplification reaction mixture volume; and
optionally, an amount of heparin equivalent to 2 units to 50 units heparin per mL of whole blood, plasma, or serum in an amplification reaction mixture.

3. The composition of claim 1 or 2 comprising trehalose in an amount of
(i) 0.1 to 1.0 M; 0.2 M to 1.0 M; 0.3 M to 0.9 M; 0.4 to 0.8 M; or 0.5 M to 0.7 M per amplification reaction mixture volume; or
(ii) 0.6 M trehalose per amplification reaction mixture volume.

4. The composition of any one of claims 1-3 comprising carnitine in an amount of:
(i) 0.1 M to 1.5 M; 0.1 M to 1.4 M; 0.1 M to 1.3 M; 0.1 Mto 1.2 M; 0.1 M to 1.1 M; 0.1 Mto 1.0 M; 0.1 M to 1.0 M; 0.1 Mto 1.0 M; 0.2 M to 0.8 M; 0.3 M to 0.7 M; or 0.4 M to 0.6 M per amplification reaction mixture volume; or
(ii) 0.5 M carnitine per amplification reaction mixture volume.

5. The composition of any one of claims 1-4 comprising:
a non-ionic detergent in an amount of 0.01% to 8% non-ionic detergent; 0.02% to 7.5% non-ionic detergent; 0.03% to 7% non-ionic detergent; 0.04% to 6.5% non-ionic detergent; 0.05% to 6% non-ionic detergent; 0.06% to 5.5% non-ionic detergent; 0.07% to 5% non-ionic detergent; 0.08% to 4.5% non-ionic detergent; 0.09% to 4% non-ionic detergent; 0.1% to 3.5% non-ionic detergent; 0.1% to 3% non-ionic detergent; 0.1% to 2.5% non-ionic detergent; 0.1% to 2% non-ionic detergent; 0.1% to 1.5% non-ionic detergent; 0.1% to 1.4% non-ionic detergent; 0.1 % to 1.3% non-ionic detergent; 0.1 % to 1.2%; 0.1 % to 1.1%; 0.2% to 1.0%; 0.3% to 0.9%; or 0.4% to 0.8% per amplification reaction mixture volume;
wherein, optionally, the non-ionic detergent is selected from the group consisting of:
(i) ethoxylated fatty alcohol ethers, lauryl ethers, ethoxylated alkyl phenols, octylphenoxy polyethoxy ethanol compounds, modified oxyethylated straight-chain alcohols, oxypropylated straight-chain alcohols, polyethylene glycol monooleate compounds, polysorbate compounds, and phenolic fatty alcohol ethers; or
(ii) Brij-58, NP-40, Nonidet P-40, Igepal CA-630, Brij-58, Tween-20, NP-40, and Triton X-100;
wherein, optionally:
if the non-ionic detergent is a polyoxyethylene cetyl ether, then the polyoxyethylene cetyl ether is at a concentration of 0.01% to 4.0%; 0.02% to 1.0%; 0.03% to 0.5%; or 0.04% to 0.2% per amplification reaction mixture volume; or
if the non-ionic detergent is a nonyl phenoxylpolyethoxylethanol, then the nonyl phenoxylpolyethoxylethanol at a concentration of 0.1% to 1.1%; 0.2% to 1.0%; 0.3% to 0.9%; or 0.4% to 0.8% per amplification reaction mixture volume.

6. The composition of any one of claims 1-5 comprising:
(i) heparin in an amount of equivalent to 2 units to 50 units; 5 units to 45 units, 5 units to 40 units, 5 units to 35 units, 5 units to 30 units, 5 units to 25 units, 5 units to 20 units, 5 units to 15 units, 6 units to 14 units, 7 units to 13 units, 8 units to 12 units, or 9 units to 11 units of heparin per mL of whole blood, blood fraction, plasma, or serum in an amplification reaction mixture volume; or
(ii) heparin in an amount of equivalent to 10 units of heparin per mL of whole blood, blood fraction, plasma, or serum in an amplification reaction mixture volume.

7. A method of amplifying a target nucleic acid in a polymerase chain reaction (PCR) comprising:
forming an assay mixture comprising:
a sample, the sample comprising a target nucleic acid;
at least one polymerase; and
an enhancer composition of any one of claims 1-6; and
amplifying the target nucleic acid in the assay mixture.

8. The method of claim 7 wherein the target nucleic acid comprises a DNA molecule; and, optionally, the PCR is a real-time PCR.

9. The method of claim 7 wherein:
the PCR is a reverse-transcriptase (RT) PCR;
the target nucleic acid comprises an RNA molecule; and
the assay mixture further comprises a reverse-transcriptase;
and, optionally, the RT-PCR is a real-time RT-PCR.

10. The method of any one of claims 7-9 wherein the sample comprises a heterogeneous mixture of nucleic acid targets, the heterogeneous mixture comprising:
(i) varying G+C content, such as 30% to 81% G+C content; or
(ii) one or more of endogenous DNA, endogenous RNA, exogenous DNA, or exogenous RNA, such as cellular DNA, cellular RNA, pathogen DNA, or pathogen RNA.

11. The method of any one of claims 7-10 wherein the assay mixture comprises a volume of whole blood, blood fraction, or a combination thereof; and
optionally, the blood fraction comprises plasma or serum; and
optionally, the whole blood, plasma, serum, or combination thereof is at least 1% up to 25%; at least 3% up to 20%; or at least 5% up to 15% of a total volume of the assay mixture.

12. The method of any one of claims 7-10 wherein the assay mixture comprises a volume of soil or soil extract; and, optionally:
(i) the soil or soil extract is at least 1% up to 90%; at least 1% up to 80%; at least 1% up to 70%; at least 1% up to 60%; at least 1% up to 50%; at least 1% up to 40%; at least 1 % up to 30%; at least 1 % up to 20%; or at least 1 % up to 10% of a total volume of the assay mixture; or
(ii) the soil or soil extract comprises a humic acid; and the soil or soil extract is present in the assay mixture at a soil or soil extract equivalent amount that provides up to 25 ng of humic acid per 50 uL reaction volume; up to 20 ng of humic acid per 50 uL reaction volume; or up to 10 ng of humic acid per 50 uL reaction volume.

13. The method of any one of claims 7-12 wherein the assay mixture comprises at least one dye, such as at least one fluorescent dye, such as at least one dye selected from the group consisting of SYBR Green, Ethidium Bromide, PICO, TOTO, YOYO or LC Green; and, optionally, the dye is present in the assay mixture at least 0.5X up to 256X, up to 128X, up to 64X, or up to 32X, where X is a manufacturer unit for concentration.

14. The method of any one of claims 7-13, wherein the assay mixture comprises at least one DNA polymerase; and, optionally:
(i) the at least one polymerase comprises a dye-resistant polymerase activity, blood-resistant polymerase activity, or soil-resistant polymerase activity, or a combination thereof;
(ii) the at least one polymerase is selected from the group consisting of OmniTaq, Omni Klentaq, Omni Klentaq-LA, wild type Taq; FastStart Taq; JumpStart Taq; HotStart Plus Taq; AmpliTaq Gold, KlenTaq, FL-12, FL-10, and KT-12; or
(iii) the assay mixture comprises at least two polymerases, such as OmniTaq and Omni Klentaq.

15. The method of any one of claims 7-11 or 14, wherein:
the sample comprises whole blood, a blood fraction, or a combination thereof; and
the enhancer composition comprises an amount of heparin effective to (i) increase efficiency of PCR or (ii) reduce or eliminate an inhibitory effect of the whole blood, blood fraction, or combination thereof on PCR.

16. The method of claim 15 wherein:
(i) the blood fraction is selected from the group consisting of plasma and serum; and
(ii) the amount of heparin is effective to reduce or eliminate the inhibitory effect of the plasma or serum on PCR.

17. The method of claim 15 or claim 16 wherein the amount of heparin is:
(i) equivalent to 2 units to 50 units; 5 units to 45 units, 5 units to 40 units, 5 units to 35 units, 5 units to 30 units, 5 units to 25 units, 5 units to 20 units, 5 units to 15 units, 6 units to 14 units, 7 units to 13 units, 8 units to 12 units, or 9 units to 11 units of heparin per mL of whole blood, blood fraction, plasma, serum, or combination thereof in the assay mixture; or
(ii) equivalent to 10 units of heparin per mL of whole blood, blood fraction, plasma, serum, or combination thereof in the assay mixture.

## Patentansprüche

1. Zusammensetzung zur Verstärkung der Nukleinsäureamplifikation, umfassend:
Trehalose;
Carnitin;
ein nicht-ionisches Detergens; und
wahlweise Heparin.

2. Zusammensetzung nach Anspruch 1, umfassend:
0,1 bis 1,0 M D-(+)-Trehalose pro Amplifikationsreaktionsmischungsvolumen;
0,1 bis 1,5 M L-Carnitin pro Amplifikationsreaktionsmischungsvolumen;
0,01 % bis 8 % nicht-ionisches Detergens pro Amplifikationsreaktionsmischungsvolumen und
wahlweise eine Menge Heparin entsprechend 2 Einheiten bis 50 Einheiten Heparin pro ml Vollblut, Plasma oder Serum in einer Amplifikationsreaktionsmischung.

3. Zusammensetzung nach Anspruch 1 oder 2, umfassend Trehalose in einer Menge von
(i) 0,1 bis 1,0 M; 0,2 M bis 1,0 M; 0,3 M bis 0,9 M; 0,4 bis 0,8 M oder 0,5 M bis 0,7 M pro Amplifikationsreaktionsmischungsvolumen oder
(ii) 0,6 M Trehalose pro Amplifikationsreaktionsmischungsvolumen.

4. Zusammensetzung nach einem der Ansprüche 1-3, umfassend Carnitin in einer Menge von:
(i) 0,1 M bis 1,5 M; 0,1 M bis 1,4 M; 0,1 M bis 1,3 M; 0,1 M bis 1,2 M; 0,1 M bis 1,1 M; 0,1 M bis 1,0 M; 0,1 M bis 1,0 M; 0,1 M bis 1,0 M; 0,2 M bis 0,8 M; 0,3 M bis 0,7 M oder 0,4 M bis 0,6 M pro Amplifikationsreaktionsmischungsvolumen oder
(ii) 0,5 M Carnitin pro Amplifikationsreaktionsmischungsvolumen.

5. Zusammensetzung nach einem der Ansprüche 1-4, umfassend:
ein nicht-ionisches Detergens in einer Menge von 0,01 % bis 8 % nicht-ionisches Detergens; 0,02 % bis 7,5 % nicht-ionisches Detergens; 0,03 % bis 7 % nicht-ionisches Detergens; 0,04 % bis 6,5 % nicht-ionisches Detergens; 0,05 % bis 6 % nicht-ionisches Detergens; 0,06 % bis 5,5 % nicht-ionisches Detergens; 0,07 % bis 5 % nicht-ionisches Detergens; 0,08 % bis 4,5 % nicht-ionisches Detergens; 0,09 % bis 4 % nicht-ionisches Detergens; 0,1 % bis 3,5 % nicht-ionisches Detergens; 0,1 % bis 3 % nicht-ionisches Detergens; 0,1 % bis 2,5 % nicht-ionisches Detergens; 0,1 % bis 2 % nicht-ionisches Detergens; 0,1 % bis 1,5 % nicht-ionisches Detergens; 0,1 % bis 1,4 % nicht-ionisches Detergens; 0,1 % bis 1,3 % nicht-ionisches Detergens; 0,1 % bis 1,2 %; 0,1 % bis 1,1 %; 0,2 % bis 1,0 %; 0,3 % bis 0,9 % oder 0,4 % bis 0,8 % pro Amplifikationsreaktionsmischungsvolumen;
wobei wahlweise das nicht-ionische Detergens ausgewählt ist aus der Gruppe bestehend aus:
(i) ethoxylierten Fettalkoholethern, Laurylethern, ethoxylierten Alkylphenolen, Octylphenoxypolyethoxyethanol-Verbindungen, modifizierten oxyethylierten geradkettigen Alkoholen, oxypropylierten geradkettigen Alkoholen, Polyethylenglykolmonooleat-Verbindungen, Polysorbat-Verbindungen und Phenolfettalkoholether oder
(ii) Brij-58, NP-40, Nonidet P-40, Igepal CA-630, Brij-58, Tween-20, NP-40 und Triton X-100;
wobei, wahlweise:
wenn das nicht-ionische Detergens ein Polyoxyethylencetylether ist, dann der Polyoxyethylencetylether eine Konzentration von 0,01 % bis 4,0 %; 0,02 % bis 1,0 %; 0,03 % bis 0,5 % oder 0,04 % bis 0,2 % pro Amplifikationsreaktionsmischungsvolumen aufweist; oder
wenn das nicht-ionische Detergens ein Nonylphenoxylpolyethoxylethanol ist, dann das Nonylphenoxylpolyethoxylethanol eine Konzentration von 0,1 % bis 1,1 %; 0,2 % bis 1,0 %; 0,3 % bis 0,9 % oder 0,4 % bis 0,8 % pro Amplifikationsreaktionmischungsvolumen aufweist.

6. Zusammensetzung nach einem der Ansprüche 1-5, umfassend:
(i) Heparin in einer Menge äquivalent zu 2 Einheiten bis 50 Einheiten; 5 Einheiten bis 45 Einheiten, 5 Einheiten bis 40 Einheiten, 5 Einheiten bis 35 Einheiten, 5 Einheiten bis 30 Einheiten, 5 Einheiten bis 25 Einheiten, 5 Einheiten bis 20 Einheiten, 5 Einheiten bis 15 Einheiten, 6 Einheiten bis 14 Einheiten, 7 Einheiten bis 13 Einheiten, 8 Einheiten bis 12 Einheiten oder 9 Einheiten bis 11 Einheiten Heparin pro ml Vollblut, Blutfraktion, Plasma oder Serum in einem Amplifikationsreaktionsmischungsvolumen oder
(ii) Heparin in einer Menge äquivalent zu 10 Einheiten Heparin pro ml Vollblut, Blutfraktion Plasma oder Serum in einem Amplifikationsreaktionsmischungsvolumen.

7. Verfahren zur Amplifizierung einer Zielnukleinsäure in einer Polymerase-Kettenreaktion (PCR), umfassend:
Bilden einer Assaymischung, umfassend:
eine Probe, wobei die Probe eine Zielnukleinsäure umfasst; mindestens eine Polymerase und
eine Enhancer-Zusammensetzung nach einem der Ansprüche 1-6 und Amplifizieren der Zielnukleinsäure in der Assaymischung.

8. Verfahren nach Anspruch 7, wobei die Zielnukleinsäure ein DNA-Molekül umfasst und wahlweise die PCR eine Echtzeit-PCR ist.

9. Verfahren nach Anspruch 7, wobei:
die PCR eine Reverse-Transkriptase (RT)-PCR ist;
die Zielnukleinsäure ein RNA-Molekül umfasst und
die Assaymischung ferner eine reverse Transkriptase umfasst;
und, wahlweise, die RT-PCR eine Echtzeit-RT-PCR ist.

10. Verfahren nach einem der Ansprüche 7-9, wobei die Probe eine heterogene Mischung von Nukleinsäurezielen umfasst, die heterogene Mischung umfassend:
(i) variierenden G + C-Gehalt, wie etwa 30 % bis 81 % G + C-Gehalt; oder
(ii) eine oder mehrere endogene DNA, endogene RNA, exogene DNA oder exogene RNA, wie etwa zelluläre DNA, zelluläre RNA, Pathogen-DNA oder Pathogen-RNA.

11. Verfahren nach einem der Ansprüche 7-10, wobei die Assaymischung ein Volumen Vollblut, Blutfraktion oder einer Kombination davon umfasst und
wahlweise die Blutfraktion Plasma oder Serum umfasst und
wahlweise das Vollblut, Plasma, Serum davon oder eine Kombination mindestens 1 % bis 25 %; mindestens 3 % bis 20 % oder mindestens 5 % bis 15 % des Gesamtvolumens der Assaymischung beträgt.

12. Verfahren nach einem der Ansprüche 7-10, wobei die Assaymischung ein Volumen Erde oder Erdextrakt umfasst; und wahlweise:
(i) die Erde oder der Erdextrakt mindestens 1 % bis 90 %; mindestens 1 % bis 80 %; mindestens 1 % bis zu 70 %; mindestens 1 % bis 60 %; mindestens 1 % bis 50 %; mindestens 1 % bis 40 %; mindestens 1 % bis 30 %; mindestens 1 % bis 20 % oder mindestens 1 % bis 10 % des Gesamtvolumens der Assaymischung beträgt oder
(ii) die Erde oder der Erdextrakt eine Huminsäure umfasst; und die Erde oder der Erdextrakt in der Assaymischung in einer Menge Erde oder Erdextrakt äquivalent der Menge vorhanden ist, die bis zu 25 ng Huminsäure pro 50 µl Reaktionsvolumen; bis zu 20 ng Huminsäure pro 50 µl Reaktionsvolumen oder bis zu 10 ng Huminsäure pro 50 µl Reaktionsvolumen bereitstellt.

13. Verfahren nach einem der Ansprüche 7-12, wobei die Assaymischung mindestens einen Farbstoff umfasst, wie etwa mindestens einen Fluoreszenzfarbstoff, wie etwa mindestens einen Farbstoff ausgewählt aus der Gruppe bestehend aus SYBR Green, Ethidiumbromid, PICO, TOTO, YOYO oder LC-Grün; und wahlweise der Farbstoff in der Assaymischung mindestens 0,5X bis 256X, bis 128X bis 64X oder bis 32X vorhanden ist, wobei X eine Herstellereinheit für Konzentration ist.

14. Verfahren nach einem der Ansprüche 7-13, wobei die Assaymischung mindestens eine DNA-Polymerase umfasst; und wahlweise:
(i) die mindestens eine Polymerase eine farbstoffresistente Polymerase-Aktivität, blutresistente Polymerase-Aktivität oder erdresistente Polymerase-Aktivität oder eine Kombination davon umfasst;
(ii) die mindestens eine Polymerase ausgewählt ist aus der Gruppe bestehend aus OmniTaq, Omni Klentaq, Omni Klentaq-LA, Wildtyp-Taq; Faststart Taq; Jumpstart Taq; HotStart Plus-Taq; AmpliTaq Gold, KlenTaq, FL-12, FL-10 und KT-12 oder
(iii) die Assaymischung mindestens zwei Polymerasen umfasst, wie etwa OmniTaq und Omni Klentaq.

15. Verfahren nach einem der Ansprüche 7-11 oder 14, wobei:
die Probe Vollblut, eine Blutfraktion oder eine Kombination davon umfasst;
und
die Enhancer-Zusammensetzung eine Menge Heparin umfasst, die wirksam ist, um (i) die Effizienz der PCR zu erhöhen, oder (ii) eine hemmende Wirkung des Vollbluts, der Blutfraktion oder einer Kombination davon auf PCR zu reduzieren oder zu eliminieren.

16. Verfahren nach Anspruch 15, wobei:
(i) die Blutfraktion ausgewählt ist aus der Gruppe bestehend aus Plasma und Serum; und
(ii) die Menge Heparin wirksam ist, die hemmende Wirkung des Plasmas oder Serums auf PCR zu reduzieren oder zu eliminieren.

17. Verfahren nach Anspruch 15 oder Anspruch 16, wobei die Menge von Heparin Folgendes ist:
(i) äquivalent zu 2 Einheiten bis 50 Einheiten; 5 Einheiten bis 45 Einheiten, 5 Einheiten bis 40 Einheiten, 5 Einheiten bis 35 Einheiten, 5 Einheiten bis 30 Einheiten, 5 Einheiten bis 25 Einheiten, 5 Einheiten bis 20 Einheiten, 5 Einheiten bis 15 Einheiten, 6 Einheiten bis 14 Einheiten, 7 Einheiten bis 13 Einheiten, 8 Einheiten bis 12 Einheiten oder 9 Einheiten bis 11 Einheiten Heparin pro ml Vollblut, Blutfraktion, Plasma, Serum oder Kombination davon in der Assaymischung; oder
(ii) äquivalent zu 10 Einheiten Heparin pro ml Vollblut, Blutfraktion, Plasma, Serum oder eine Kombination davon in der Assaymischung.

## Revendications

1. Composition d'amélioration de l'amplification d'acides nucléiques, comprenant :
du tréhalose ;
de la carnitine ;
un détergent non ionique ; et
éventuellement de l'héparine.

2. Composition selon la revendication 1, comprenant :
0,1 à 1,0 M de D-(+)-tréhalose par volume de mélange réactionnel d'amplification ;
0,1 à 1,5 M de L-carnitine par volume de mélange réactionnel d'amplification ;
0,01 % à 8 % de détergent non ionique par volume de mélange réactionnel d'amplification ; et
éventuellement, une quantité d'héparine équivalente à 2 unités à 50 unités d'héparine par millilitre de sang entier, de plasma ou de sérum dans un mélange de réaction d'amplification.

3. Composition selon la revendication 1 ou 2, comprenant du tréhalose à hauteur de
(i) 0,1 à 1,0 M ; 0,2 M à 1,0 M ; 0,3 M à 0,9 M ; 0,4 à 0,8 M ; ou 0,5 M à 0,7 M par volume de mélange réactionnel d'amplification ; ou
(ii) 0,6 M de tréhalose par volume de mélange réactionnel d'amplification.

4. Composition selon l'une quelconque des revendications 1 à 3, comprenant de la carnitine à hauteur de :
(i) 0,1 M à 1,5 M ; 0,1 M à 1,4 M ; 0,1 M à 1,3 M ; 0,1 M à 1,2 M ; 0,1 M à 1,1 M ; 0,1 M à 1,0 M ; 0,1 M à 1,0 M ; 0,1 M à 1,0 M ; 0,2 M à 0,8 M ; 0,3 M à 0,7 M ; ou 0,4 M à 0,6 M par volume de mélange réactionnel d'amplification ; ou
(ii) 0,5 M de carnitine par volume de mélange réactionnel d'amplification.

5. Composition selon l'une quelconque des revendications 1 à 4, comprenant :
un détergent non ionique à hauteur de 0,01 % à 8 % de détergent non ionique ; de 0,02 % à 7,5 % de détergent non ionique ; de 0,03 % à 7 % de détergent non ionique ; de 0,04 % à 6,5 % de détergent non ionique ; de 0,05 % à 6 % de détergent non ionique ; de 0,06 % à 5,5 % de détergent non ionique ; de 0,07 % à 5 % de détergent non ionique ; de 0,08 % à 4,5 % de détergent non ionique ; de 0,09 % à 4 % de détergent non ionique ; de 0,1 % à 3,5 % de détergent non ionique ; de 0,1 % à 3 % de détergent non ionique ; de 0,1 % à 2,5 % de détergent non ionique ; de 0,1 % à 2 % de détergent non ionique ; de 0,1 % à 1,5 % de détergent non ionique ; de 0,1 % à 1,4 % de détergent non ionique ; de 0,1 % à 1,3 % de détergent non ionique ; de 0,1 % à 1,2 % ; de 0,1 % à 1,1 % ; de 0,2 % à 1,0 % ; de 0,3 % à 0,9 % ; ou de 0,4 % à 0,8 % par volume de mélange réactionnel d'amplification ;
dans laquelle, en option, le détergent non ionique est choisi dans l'ensemble constitué des éléments suivants :
(i) des éthers d'alcools gras éthoxylés, des lauryl-éthers, des alkylphénols éthoxylés, des composés d'octylphénoxy-polyéthoxyéthanol, des alcools à chaîne linéaire oxyéthylés modifiés, des alcools à chaîne linéaire oxypropylés, des composés de mono-oléate de polyéthylène glycol, des composés de polysorbate et des éthers d'alcools gras phénoliques ; ou
(ii) Brij-58, NP-40, Nonidet P-40, Igepal CA-630, Brij-58, Tween-20, NP-40 et Triton X-100 ;
où en option :
si le détergent non ionique est un éther cétylique de polyoxyéthylène, alors l'éther cétylique de polyoxyéthylène est à une concentration de 0,01 % à 4,0 % ; de 0,02 % à 1,0 % ; de 0,03 % à 0,5 % ; ou de 0,04 % à 0,2 % par volume de mélange réactionnel d'amplification ; ou
si le détergent non ionique est un nonyl phénoxylpolyéthoxyléthanol, alors le nonyl-phénoxylpolyéthoxyléthanol est à une concentration de 0,1 % à 1,1 % ; de 0,2 % à 1,0 % ; de 0,3 % à 0,9 % ; ou de 0,4 % à 0,8 % par volume de mélange réactionnel d'amplification.

6. Composition selon l'une quelconque des revendications 1 à 5, comprenant :
(i) de l'héparine à hauteur de l'équivalent de 2 unités à 50 unités ; 5 unités à 45 unités ; 5 unités à 40 unités ; 5 unités à 35 unités ; 5 unités à 30 unités ; 5 unités à 25 unités ; 5 unités à 20 unités ; 5 unités à 15 unités ; 6 unités à 14 unités ; 7 unités à 13 unités ; 8 unités à 12 unités ou 9 unités à 11 unités d'héparine par millilitre de sang entier, de fraction de sang, de plasma ou de sérum dans un volume de mélange réactionnel d'amplification ; ou
(ii) de l'héparine à hauteur d'un équivalent de 10 unités d'héparine par millilitre de sang entier, de fraction de sang, de plasma ou de sérum dans un volume de mélange réactionnel d'amplification.

7. Procédé d'amplification d'un acide nucléique cible dans une amplification en chaîne par polymérase (PCR) comprenant :
la formation d'un mélange d'essai comprenant :
un échantillon, l'échantillon comprenant un acide nucléique cible :
au moins une polymérase ; et
une composition d'amélioration selon l'une quelconque des revendications 1 à 6 ; et
l'amplification de l'acide nucléique cible dans le mélange d'essai.

8. Procédé selon la revendication 7, dans lequel l'acide nucléique cible contient une molécule d'ADN ; et où éventuellement la PCR est une PCR en temps réel.

9. Procédé selon la revendication 7, dans lequel :
la PCR est une PCR à transcriptase inverse (RT) ;
l'acide nucléique cible comprend une molécule d'ARN ; et
le mélange d'essai comprend en outre une transcriptase inverse ;
et éventuellement, la RT-PCR est une RT-PCR en temps réel.

10. Procédé selon l'une quelconque des revendications 7 à 9, dans lequel l'échantillon contient un mélange hétérogène de cibles d'acides nucléiques, le mélange hétérogène comprenant :
(i) la variation de la teneur en G+C, telle que de 30 % à 81 % de teneur en G+C ;
ou
(ii) au moins un ADN endogène, un ARN endogène, un ADN exogène ou un ARN exogène, tel qu'un ADN cellulaire, un ARN cellulaire, un ADN de pathogène ou un ARN de pathogène.

11. Procédé selon l'une quelconque des revendications 7 à 10, dans lequel le mélange d'essai comprend un volume de sang entier, de fraction de sang ou d'une combinaison des deux ; et
en option, la fraction de sang contient du plasma ou du sérum ; et
en option, le sang entier, le plasma, le sérum ou leur combinaison représente au moins 1 % à 25 % ; au moins 3 % à 20 % ; ou au moins 5 % à 15 % d'un volume total du mélange d'essai.

12. Procédé selon l'une quelconque des revendications 7 à 10, dans lequel le mélange d'essai comprend un volume de sol ou d'extrait de sol ; et éventuellement :
(i) le sol ou l'extrait de sol représente au moins 1 % et jusqu'à 90 % ; au moins 1 % et jusqu'à 80 % ; au moins 1 % et jusqu'à 70 % ; au moins 1 % et jusqu'à 60 % ; au moins 1 % et jusqu'à 50 % ; au moins 1 % et jusqu'à 40 % ; au moins 1 % et jusqu'à 30 % ; au moins 1 % et jusqu'à 20 % ; ou au moins 1 % et jusqu'à 10 % d'un volume total du mélange d'essai ; ou
(ii) le sol ou l'extrait de sol contient un acide humique ; et le sol ou l'extrait de sol est présent dans le mélange d'essai en une quantité équivalente de sol ou d'extrait de sol qui procure jusqu'à 25 ng d'acide humique pour 50 µl de volume réactionnel ; jusqu'à 20 ng d'acide humique pour 50 µl de volume réactionnel ; ou jusqu'à 10 ng d'acide humique pour 50 µl de volume réactionnel.

13. Procédé selon l'une quelconque des revendications 7 à 12, dans lequel le mélange d'essai contient au moins un colorant, tel qu'au moins un colorant fluorescent, tel qu'au moins un colorant choisi dans l'ensemble constitué du SYBR Green, du bromure d'éthidium, du PICO, du TOTO, du YOYO ou du LC Green ; et en option, le colorant est présent dans le mélange d'essai au moins 0,5X jusqu'à 256X, jusqu'à 128X, jusqu'à 64X ou jusqu'à 32X, où X représente une unité de fabricant pour la concentration.

14. Procédé selon l'une quelconque des revendications 7 à 13, dans lequel le mélange d'essai contient au moins une ADN polymérase ; et en option :
(i) la ou les polymérases présentent une activité de polymérase résistant au colorant, une activité de polymérase résistant au sang ou une activité de polymérase résistant au sol, ou une combinaison de celles-ci ;
(ii) la ou les polymérases sont choisies dans l'ensemble constitué d'OmniTaq, d'Omni Klentaq, d'Omni Klentaq-LA, de Taq de type sauvage ; de FastStart Taq ; de JumpStart Taq ; de HotStart Plus Taq ; d'AmpliTaq Gold, de KlenTaq, de FL-12, de FL-10 et de KT-12 ; ou
(iii) le mélange d'essai contient au moins deux polymérases, telles que l'OmniTaq et l'Omni Klentaq.

15. Procédé selon l'une quelconque des revendications 7 à 11 ou 14, dans lequel :
l'échantillon contient du sang entier, une fraction de sang ou une combinaison de ceux-ci ; et
la composition d'amélioration contient une quantité d'héparine efficace pour (i) augmenter l'efficacité de la PCR ou (ii) réduire ou éliminer un effet d'inhibition du sang entier, de la fraction de sang ou de leur combinaison sur la PCR.

16. Procédé selon la revendication 15, dans lequel :
(i) la fraction de sang est choisie dans l'ensemble constitué de plasma et de sérum ;
et
(ii) la quantité d'héparine est efficace pour réduire ou pour éliminer l'effet d'inhibition du plasma ou du sérum sur la PCR.

17. Procédé selon la revendication 15 ou 16, dans lequel la quantité d'héparine est :
(i) équivalente à 2 unités à 50 unités ; 5 unités à 45 unités, 5 unités à 40 unités, 5 unités à 35 unités, 5 unités à 30 unités, 5 unités à 25 unités, 5 unités à 20 unités, 5 unités à 15 unités, 6 unités à 14 unités, 7 unités à 13 unités, 8 unités à 12 unités, ou 9 unités à 11 unités d'héparine par millilitre de sang entier, d'une fraction de sang, de plasma, de sérum ou d'une combinaison de ceux-ci dans le mélange d'essai ; ou
(ii) équivalente à 10 unités d'héparine par millilitre de sang entier, d'une fraction de sang, de plasma, de sérum ou d'une combinaison de ceux-ci dans le mélange d'essai.


### SEQUENCE LISTING

<110> DNA Polymerase Technology Inc.
   Zhang, Zhian
   Kermekchiev, Milko
<120> COMPOSITIONS FOR IMPROVING NUCLEIC ACID AMPLIFICATION
<130> 60019630-0070
<150> US 61/201,052
   <151> 2008-12-05
<150> US 61/160,606
   <151> 2009-03-16
<160> 20
<170> PatentIn version 3.5
<210> 1
   <211> 33
   <212> DNA
   <213> Artificial Sequence
<220>
   <223> PCR primer
<400> 1
   gcagcggcag gaccagcccc aagatgacta tct 33
<210> 2
   <211> 33
   <212> DNA
   <213> Artificial Sequence
<220>
   <223> PCR primer
<400> 2
   tggaacaaga tggattatca agtgtcaagt cca 33
<210> 3 <211> 25
   <212> DNA
   <213> Artificial Sequence
<220>
   <223> PCR primer
<400> 3
   tccggccgcg ccaccgccac cctca 25
<210> 4
   <211> 25
   <212> DNA
   <213> Artificial Sequence
<220>
   <223> PCR primer
<400> 4
   accgccagag cgcccagccc gcgca 25
<210> 5
   <211> 25
   <212> DNA
   <213> Artificial Sequence
<220>
   <223> PCR primer
<400> 5
   tcacccacac tgtgcccatc tacga 25
<210> 6
   <211> 25
   <212> DNA
   <213> Artificial Sequence
<220>
   <223> PCR primer
<400> 6
   cagcggaacc gctcattgcc aatgg 25
<210> 7
   <211> 21
   <212> DNA
   <213> Artificial Sequence
<220>
   <223> PCR primer
<400> 7
   atcaccagag gcaagacacc c 21
<210> 8
   <211> 21
   <212> DNA
   <213> Artificial Sequence
<220>
   <223> PCR primer
<400> 8
   accaatatca aagaacgacg c 21
<210> 9
   <211> 23
   <212> DNA
   <213> Artificial Sequence
<220>
   <223> PCR primer
<400> 9
   atgccccaac cattgaagca atc 23
<210> 10
   <211> 23
   <212> DNA
   <213> Artificial Sequence
<220>
   <223> PCR primer
<400> 10
   acgtccattg attttgctgg atg 23
<210> 11
   <211> 20
   <212> DNA
   <213> Artificial Sequence
<220>
   <223> PCR primer
<400> 11
   ccttctgttc tgtgcagtgg 20
<210> 12
   <211> 18
   <212> DNA
   <213> Artificial Sequence
<220>
   <223> PCR primer
<400> 12
   cggactccgg gactacag 18
<210> 13
   <211> 20
   <212> DNA
   <213> Artificial Sequence
<220>
   <223> PCR primer
<400> 13
   ccgttttcac gtgtgtgtgt 20
<210> 14
   <211> 20
   <212> DNA
   <213> Artificial Sequence
<220>
   <223> PCR primer
<400> 14
   ttccttctcc ctcctggtct 20
<210> 15
   <211> 19
   <212> DNA
   <213> Artificial Sequence
<220>
   <223> PCR primer
<400> 15
   aggggaagga agcaggact 19
<210> 16
   <211> 18
   <212> DNA
   <213> Artificial Sequence
<220>
   <223> PCR primer
<400> 16
   cagctcagcc aggctctc 18
<210> 17
   <211> 25
   <212> DNA
   <213> Artificial Sequence
<220>
   <223> PCR primer
<400> 17
   agaagatatc agacgatcca caatc 25
<210> 18
   <211> 25
   <212> DNA
   <213> Artificial Sequence
<220>
   <223> PCR primer
<400> 18
   gtagaacgac gccagaataa gaata 25
<210> 19
   <211> 21
   <212> DNA
   <213> Artificial Sequence
<220>
   <223> PCR primer
<400> 19
   cttctaaccg aggtcgaaac g 21
<210> 20
   <211> 24
   <212> DNA
   <213> Artificial Sequence
<220>
   <223> PCR primer
<400> 20
   agggcatttt ggacaaarcg tcta 24